# EUROPEAN PATENT APPLICATION

(11) **EP 4 382 136 A1**
(43) Date of publication of application: **12.06.2024**
(21) Application number: 22212471.1
(22) Date of filing: 09.12.2022
(51) Int. Cl.: A61K 49/00, A61K 49/10, C07B 59/00, A61K 51/04

(54) **CYCLEN BASED COMPOUNDS AND THEIR GD(III) COMPLEXES FOR USE AS MULTIMODAL PET/MRI CONTRAST AGENTS**

(71) Applicant: Ustav organicke chemie a biochemie AV CR, v.v.i., 166 10 Praha 6 (CZ)
(72) Inventor: Polasek, Miloslav, 16000 Praha 6 (CZ); Kretschmer, Jan, 14000 Praha 4 (CZ)
(74) Representative: Harber IP s.r.o.

(57) **Abstract**

The present invention relates to the use of cyclen based compound of general formula (I) 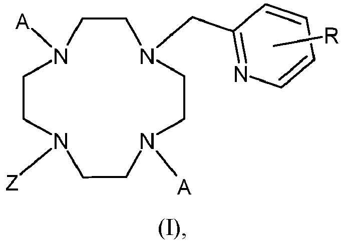 wherein
Ris NO₂ or F;
A is independently selected from -CH₂COOH; -CH(CH₃)COOH; ; -CH((CH₂)ₙCH₃)COOH, wherein n is an integer in the range of from 1 to 3; and -CH₂P(=O)(OH)Ph;
Z is H or A;
for preparation of a multimodal PET/MRI contrast agents. The invention also relates to Gd(III) complexes of compound of general formula (I), wherein R is ¹⁸F, as multimodal PET/MRI contrast agents.

## Description

### Technical field

This invention relates to cyclen based compounds, their Gd(III) complexes, and their use as multimodal PET/MRI contrast agents, containing ¹⁸F and Gd³⁺ within one complex molecule.

### Background art

Magnetic Resonance Imaging (MRI) and Positron Emission Tomography (PET) are among the most powerful tools in clinical diagnostic imaging. The information that these techniques provide is to a great degree complementary. The latest and most advanced instruments combine advantages of both methods into a single hybrid PET/MRI technology.

MRI is appreciated for providing an excellent anatomical image of soft tissue with high resolution. It is based on the magnetic resonance signal of ¹H nuclei in water molecules, which constitute about 60 - 70 % of body weight. Organs can be easily distinguished in MRI owing to different water content or relaxation rate of the magnetic resonance (MR) signal. In cases when the native contrast is insufficient, exogenous contrast agents can be intravenously applied. These are mostly based on gadolinium(III) chelates, known as gadolinium-based contrast agents (GBCAs). Typical examples are the chelates of the macrocyclic ligand DOTA (1,4,7,10-tetraazacyclododecane-1,4,7,10-tetraacetic acid) and the open-chain ligand DTPA (diethylenetriaminepentaacetic acid). The purpose of the paramagnetic Gd(III) ion is to induce relaxation of nearby ¹H nuclei (of water), while the ligand serves to mask toxicity of this metal ion. In general, the macrocyclic DOTA-like chelators are known to provide Gd(III) chelates with much higher kinetic inertness compared to the acyclic DTPA-like chelators. High kinetic inertness is desirable for *in-vivo* applications, because it prevents the toxic Gd(III) ion from escaping from the chelate. Thus, macrocyclic chelators are preferred. Currently used GBCAs are non-specific compounds, i.e. they do not bind to a specific molecular target. Yet, they provide important diagnostic information based on differences in diffusion and perfusion characteristics of the tissue, which are often linked to pathological changes. Resulting differences in biodistribution of the GBCAs are visualized in MRI and reveal the pathologies.

PET is valued for providing specific information about molecular targets with very high sensitivity. This technique is based on administration of tracer molecules labelled with a positron-emitting radionuclide. When the radionuclide decays, the emitted positron annihilates with a nearby electron, creating two gamma photons that travel in opposite direction (180 degrees apart). An external array of detectors registers both photons and reveals their point of origin, allowing to construct an image of the tracer molecules in the body. Thanks to this photon-counting principle, PET is a very sensitive and quantitative technique. Extremely low amounts of the tracer molecules are needed for detection, allowing targeting and imaging of low-abundance molecular targets. A disadvantage is the lack of anatomical image. For this reason, PET is combined with computed tomography (CT) or (more recently) with MRI that both provide the anatomical image for reference. Numerous PET tracers are clinically approved or in development, using various PET radionuclides, such as ¹¹C, ¹³N, ¹⁸F, ⁶⁴Cu, ⁶⁸Ga, ⁸⁹Zr and others. Among these, ¹⁸F is the most widely used, because it is cost-effectively prepared in large quantities by cyclotron irradiation of ¹⁸O-enriched water. With a moderate half-life of 110 minutes, ¹⁸F is amenable for synthesis of a broad range of small-molecular organic tracer molecules, including the most common clinical PET tracer, [¹⁸F]FDG (fluorodeoxyglucose). Combined PET/MRI instruments provide significant advantages over separate PET and MRI and other imaging techniques. Mainly, examination with PET/MRI decreases the radiation burden to the patient compared to PET/CT, while providing superior anatomical image of soft tissue. Obtaining temporally and spatially matched images from both modalities simultaneously offers opportunities for increasing diagnostic accuracy, speed and discovery of new diagnostic imaging markers. However, the current practice of PET/MRI examinations is to administer two separate contrast agents: the radioactive tracer for PET and the GBCA for MRI. These have completely independent pharmacokinetic behaviour and provide independent diagnostic information. There are no clinically approved agents that would concurrently provide the PET signal and MRI contrast from a single compound. In fact, only a few such compounds have been reported in literature so far. Combining the Gd(III) chelate with the PET radionuclide into a single stable and well-defined molecule presents significant synthetic challenges that hamper the progress. One of the earliest examples [Frullano, L. et al., Angew. Chem. Int. Ed. 2010, 49 (13), 2382-2384] reported a pH responsive PET/MRI contrast agent based on a Gd(III) chelate that was equipped with an ¹⁸F-bearing prosthetic group via a click reaction, using multi-step synthesis with only 0.6% overall yield. Later, the same group prepared a PET/MRI version of the fibrin-targeted MRI agent EP-2104R by partial dechelation of one of its four Gd(III) chelates and subsequent complexation of ⁶⁴Cu(II) [Uppal, R. et al., Radiology 2011, 258 (3), 812-820]. However, the lack of control over the decomplexation/complexation strategy yielded a mixture of positional isomers, not a single well-defined compound. Others have tried to create dendrimer-like multi-metallic structures by decorating a single ⁶⁸Ga(III) chelate with multiple Gd(III) chelates, creating rather large and complicated molecules [Kumar, A. et al., Bioconjugate Chem. 2015, 26 (3), 549-558; Notni, J. et al., Chem. Eur. J. 2013, 19 (38), 12602-12606]. Translating such complicated molecules to clinical practice is typically hampered by poor reproducibility of the synthesis.

Another challenge faced in preparing combined PET/MRI contrast agent is that the amount of radionuclide needed for the PET signal is about 9-10 orders of magnitude lower than the Gd(III) chelate needed for the MRI contrast. To match these conflicting quantity requirements, mixture of two isotopically different versions of the agent can be prepared. The agent containing the active PET isotope is present only in trace quantities needed to obtain the PET signal, while majority of the agent is a chemically identical molecule containing a stable (non-radioactive) isotope. This bulk of non-radioactive version is needed to achieve the desired MRI contrast. Since the two versions are chemically identical (only different in isotopes), they possess the same pharmacokinetic properties. However, in cases when the agent utilizes metal PET radionuclides this means co-administration of a relatively large amount of non-radioactive exogenous metal, which presents a significant metal-induced toxicity risk. From this point of view, the use of ¹⁸F is advantageous over the metal PET radionuclides. Not only is ¹⁸F widely available and cheap isotope, but it forms stable covalent C-F bonds and fluorinated compounds are generally well tolerated.

Despite of the favorable properties of the ¹⁸F isotope, challenges remain to achieve its fast and efficient incorporation into the PET/MRI agent, especially in relation to stability of the Gd(III) chelate as the source of MRI contrast. In order to supress the losses of the ¹⁸F isotope due to radioactive decay, it is desirable to connect it to the molecule ideally in the last synthetic step. Methods have been developed to achieve this for purely organic PET tracers, but the presence of Gd(III) chelates in PET/MRI agents is incompatible with many synthetic and purification conditions. For example, Gd(III) chelates are generally poorly soluble in water-free organic solvents required for the ¹⁸F labelling chemistry. They are also unstable under acidic conditions, which may be required for chromatographic or ion-exchange purification of the final products. Thus, the conditions developed for synthesis of purely organic PET tracers are not straightforwardly transferrable to chemistry of Gd(III) chelates.

The background art thus lacks compounds and methods for fast and efficient labelling of Gd(III) chelates with ¹⁸F radionuclide that would provide access to well-defined small-molecular PET/MRI agents akin to the currently clinically used MRI contrast agents.

### Disclosure of Invention

In the background art, a modest number of bimodal PET/MRI agents have been prepared, based on rather complicated bimetallic compounds or multi-step synthesis to achieve labelling with the PET isotope. These examples fail to provide a simple and synthetically easily achievable molecular PET/MRI agent akin to the current clinically used non-specific MRI contrast agents. In contrast to this, we have found that macrocyclic Gd(III) chelates bearing a pyridine arm equipped with a suitable leaving group can be conveniently labelled with the ¹⁸F-fluoride anion via a nucleophilic attack. This single-step reaction is fast and proceeds directly on the pre-formed Gd(III) chelate. There is therefore no need for additional complexation step. The macrocyclic chelator molecule is structurally closely related to DOTA, which has a long proven history of safe clinical use as an MRI contrast agent in the form of its Gd(III) chelate. Surprisingly, we found that ¹⁸F labelling is also possible by ¹⁹F to ¹⁸F isotopic exchange on the pyridine moiety of the Gd(III) chelate, but this reaction does not take place at all, or proceeds with much lower yields, if performed on the free macrocyclic chelator. This ensures that the Gd(III) chelate is labelled preferentially, leading to a cleaner desired product that contains both Gd(III) and ¹⁸F to act as a multimodal PET/MRI agent. Moreover, this synthetic approach allows to adjust the molar ratio between the PET-active (radiolabeled) version and the non-radioactive version of the agent to tune the desired PET signal and MRI contrast to the particular PET/MRI instrument and application. Thus, these compounds and the method of their radiolabeling overcome the problems of background art mentioned above.

The object of the present invention is the use in medicine, preferably the use for preparation of a multimodal PET/MRI contrast agent, of cyclen based compound of general formula (I) 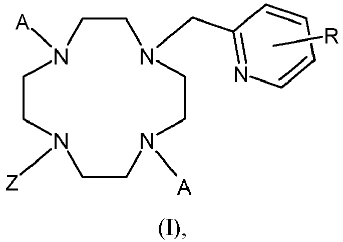 wherein
R is NO₂ or F;
A is independently selected from -CH₂COOH; -CH(CH₃)COOH; -CH((CH₂)ₙCH₃)COOH, wherein n is an integer in the range of from 1 to 3; -CH₂P(=O)(OH)Ph;
Z is H or A.

The compounds of general formula (I) can be synthetised from nitro- or fluoro-pyridine intermediates of general formula (II) 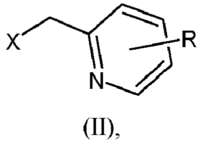 wherein
R is NO₂ or F;
and X is halogen, preferably Cl or Br.

The intermediates of general formula (II) react with cyclen-based intermediate of general formula (III) 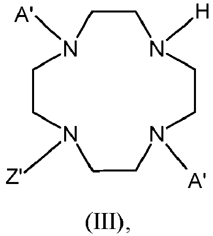 wherein
A' is A (as defined above) in which OH group is protected, preferably as methoxy, ethoxy or tert-butoxy group;
and Z' is hydrogen or A'.

The resulting precursor of general formula (IV) 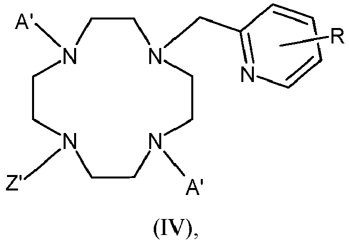 wherein Z' and A' are as defined above;
is then deprotected (the protection of OH groups is removed), preferably by using trifluoroacetic acid, giving the compound of general formula (I) as defined above.

In one preferred embodiment of cyclen based compound of general formula (I), A and Z are the same, preferably selected from -CH₂P(=O)(OH)Ph; and -CH(CH₃)COOH.

In one preferred embodiment of cyclen based compound of general formula (I), A are the same, preferably selected from -CH₂P(=O)(OH)Ph; and -CH(CH₃)COOH; and Z is hydrogen.

In one preferred embodiment, R is located in para-position in relation to the nitrogen atom of the pyridyl pendant arm. More preferably, R is F.

In one embodiment, the cyclen based compound of general formula (I) is selected from the group comprising:
2,2',2"-(10-((4-nitropyridin-2-yl)methyl)-1,4,7,10tetraazacyclododecane-1,4,7triyl)triacetic acid (**L¹**)
2,2',2"-(10-((6-nitropyridin-2-yl)methyl)-1,4,7,10tetraazacyclododecane-1,4,7triyl)triacetic acid (**L²**)
2,2',2"-(10-((3-fluoropyridin-2-yl)methyl)-1,4,7,10 tetraazacyclododecane-1,4,7 triyl)triacetic acid (**L³**)
2,2',2"-(10-((4-fluoropyridin-2-yl)methyl)-1,4,7,10 tetraazacyclododecane-1,4,7 triyl)triacetic acid (**L⁴**)
2,2',2"-(10-((5-fluoropyridin-2-yl)methyl)-1,4,7,10 tetraazacyclododecane-1,4,7 triyl)triacetic acid (**L⁵**)
2,2',2"-(10-((6-fluoropyridin-2-yl)methyl)-1,4,7,10 tetraazacyclododecane-1,4,7 triyl)triacetic acid (**L⁶**)
2,2'-(4-((4-fluoropyridin-2-yl)methyl)-1,4,7,10 tetraazacyclododecane-1,7-diyl)diacetic acid (**L⁷**)
(2*S*,2'*S*,2"*S*)-2,2',2"-(10-((3-fluoropyridin-2-yl)methyl)-1,4,7,10-tetraazacyclododecane-1,4,7-triyl)tripropionic acid (**L⁸**)
((4-((3-fluoropyridin-2-yl)methyl)-1,4,7,10-tetraazacyclododecane-1,7-diyl)bis(methylene))bis(phenylphosphinic acid) (**L⁹**)
2,2'-(4-((4-nitropyridin-2-yl)methyl)-1,4,7,10-tetraazacyclododecane-1,7-diyl)diacetic acid (**L¹⁰**)
(2*S*,2'*S*,2"*S*)-2,2',2"-(10-((4-fluoropyridin-2-yl)methyl)-1,4,7,10-tetraazacyclododecane-1,4,7-triyl)tripropionic acid (**L¹¹**)
(2*S*,2'*S*,2"*S*)-2,2',2"-(10-((4-nitropyridin-2-yl)methyl)-1,4,7,10-tetraazacyclododecane-1,4,7-triyl)tripropionic acid (**L¹²**)

The object of the present invention is also a compound of general formula (Ia) 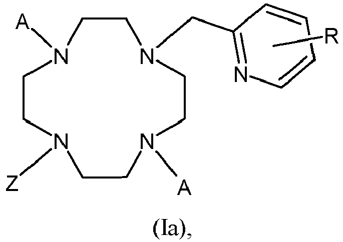 wherein R is NOz or F; preferably R is F;

A is independently selected from -CH(CH₃)COOH; -CH((CH₂)ₙCH₃)COOH, wherein n is an integer in the range of from 1 to 3; and -CH₂P(=O)(OH)Ph; and Z is H or A, preferably Z is A.

Preferably, the R substituent is located in para-position in relation to the nitrogen atom of the pyridyl pendant arm. More preferably, A is -CH(CH₃)COOH.

In the most preferred embodiment, the compound according to the present invention has the following formula (V), wherein the stereoconfiguration of all-C^{∗}H(CH₃)COOH groups is the same (either all have S configuration or all have R configuration,

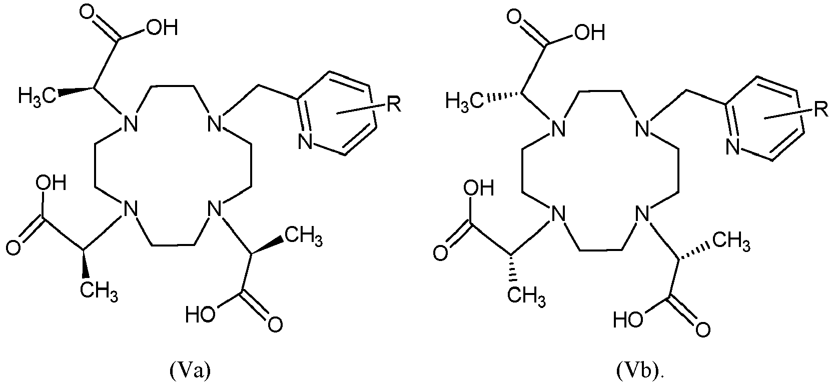

Preferably, R substituent in the compound of formula (Va) or (Vb) is located in para-position in relation to the nitrogen atom of the pyridyl pendant arm. More preferably, R is F.

Yet another object of the present invention is a coordination compound of Gd(III) ion and the cyclen based compound of general formula (I) or (Ia) as defined above, and its use in medicine for preparation of a multimodal PET/MRI contrast agent. The coordination compound can be prepared by reacting the cyclen based compound of general formula (I) or (Ia) with Gd³⁺ salt (e.g. GdCl₃), thereby forming a Gd(III) chelate of the compound of general formula (I) or (Ia). Such Gd(III) chelate is a suitable contrast agent for MRI imaging because of its high thermodynamic stability and kinetic inertness and favourable relaxivity properties.

The multimodal PET/MRI contrast agent may be prepared from the above described coordination compound of Gd(III) ion and the cyclen based compound of general formula (I) or (Ia), by radiolabelling the Gd (III) chelate with ¹⁸F. The radiolabelling may be performed either using ¹⁹F to ¹⁸F isotopic exchange on the pyridine moiety of the Gd(III) chelate, wherein R is F, or using nucleophilic aromatic substitution of nitro group with ¹⁸F, wherein R is NOz. A combination of both radiolabelling methods may also be used for Gd(III) chelates containing R being NOz, wherein a nucleophilic aromatic substitution of nitro group with ¹⁹F is performed, followed by ¹⁹F to ¹⁸F isotopic exchange. In all cases, the radiolabelling is performed on the Gd(III) chelate, not on the ligand itself. The product thus contains both Gd(III) and ¹⁸F and is suitable to act as a multimodal PET/MRI agent. Preferably, the radiolabelling is performed using a solution containing ¹⁸F⁻ produced on a cyclotron, mixed with the Gd(III) chelate at 90 °C, preferably for at least 5 minutes.

Another object of the present invention is thus a multimodal PET/MRI contrast agent of general formula (VI) 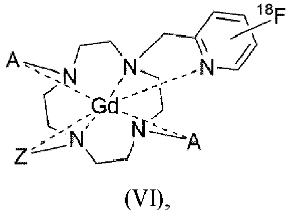
wherein A is independently selected from -CH₂COOH; -CH(CH₃)COOH; -CH((CH₂)ₙCH₃)COOH, wherein n is an integer in the range of from 1 to 3; and -CH₂P(=O)(OH)Ph;
Z is H or A.

Preferably, ¹⁸F is in para-position in relation to the nitrogen atom of the pyridyl pendant arm.

In one embodiment, the multimodal PET/MRI contrast agent has general formula (VIa) 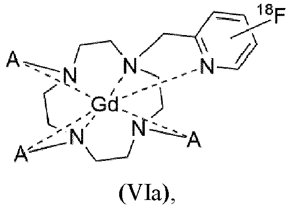 wherein A is independently selected from -CH₂COOH; -CH(CH₃)COOH; -CH((CH₂)ₙCH₃)COOH, wherein n is an integer in the range of from 1 to 3; and -CH₂P(=O)(OH)Ph; preferably, A is -CH(CH₃)COOH or -CH₂P(=O)(OH)Ph.

Preferably, if ¹⁸F is in para-position in relation to the nitrogen atom of the pyridyl pendant arm, then A is not -CH₂COOH.

In one preferred embodiment, Z is A, more preferably, A is -CH(CH₃)COOH, even more preferably, stereoconfiguration of all A (-C^{∗}H(CH₃)COOH) is the same (either all A have S configuration or all A have R configuration). Most preferably, all A have the same stereoconfiguration and ¹⁸F is in para-position in relation to the nitrogen atom of the pyridyl pendant arm.

In one embodiment, the multimodal PET/MRI contrast agent has general formula (VIb) 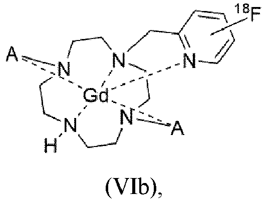 wherein A is independently selected from -CH₂COOH; -CH(CH₃)COOH; -CH((CH₂)ₙCH₃)COOH, wherein n is an integer in the range of from 1 to 3; and -CH₂P(=O)(OH)Ph; preferably, A is -CH(CH₃)COOH or -CH₂P(=O)(OH)Ph. More preferably, A is -CH(CH₃)COOH, even more preferably, stereoconfiguration of both A (-C^{∗}H(CH₃)COOH) is the same.

In the most preferred embodiment, the multimodal PET/MRI contrast agent has general formula (VI), (VIa) or (VIb), wherein ¹⁸F is in para-position in relation to the nitrogen atom of the pyridyl pendant arm.

The object of the present invention is also a method of preparing the multimodal PET/MRI contrast agent of general formula (VI) as defined above, the method comprising the following steps:
a) providing the cyclen based compound of general formula (I) as defined above;
b) forming a Gd(III) chelate of the compound of general formula (I);
c) radiolabelling the Gd (III) chelate with ¹⁸F.

In one embodiment, step b) is achieved by reacting the cyclen based compound of general formula (I) with Gd³⁺ ion, thereby forming a Gd(III) chelate of the compound of general formula (I). Preferably, the conditions of the chelation reaction are the following:
A solution containing Gd³⁺ ion in the form of salt (e.g. chloride, bromide, sulfate, nitrate, methanesulfonate, trifluoromethanesulfonate, formate, acetate, lactate, malate, citrate, 2-hydroxyisobutyrate, mandelate, diglycolate, tartarate) is mixed with a solution of the compound of general formula (I), preferably in molar ratio of Gd³⁺ ion to compound of general formula (I) in the range of from 1:0.5 to 1:100, more preferably from 1:0.7 to 1:50, even more preferably from 1:0.9 to 1:10. Concentrations of the soluble components may be selected from the concentration range permitted by solubility of such compounds in a given solvent at a given temperature, preferably in the concentration range 0.000001 - 0.5 mol/L. The solvent may be water, a water-miscible organic solvent such as methanol, ethanol, propanol, isopropanol, acetone, acetonitrile, N,N-dimethylformamide, dimethylsulfoxide, tetrahydrofurane, or a mixture thereof. An organic or inorganic base, such as LiOH, NaOH, KOH, aqueous NH₃, triethylamine, N,N-diisopropylethylamine or pyridine, is added to the reaction mixture in order to compensate for protons released during the complexation, and the complexation takes place in the solution. Preferably, from 1 to 10 molar equivalents of base are added per molecule of the compound of general formula (I). Eventually, the reaction can take place in a buffer. In such case there is no need of adding organic or inorganic base to the reaction mixture. The mixture is stirred or shaken at room temperature or elevated temperature for up to 24 hours to afford complete complexation. Preferably, the mixture is stirred or shaken at 40 °C for 15 minutes. A reasonable excess of the compound of general formula (I) may be used to accelerate the complexation and to shift the equilibrium towards formation of the chelates. The resulting Gd(III) chelate of the compound of general formula (I) may optionally be desalted and purified from excess of the compound of general formula (I) and other impurities. The purification may be achieved, for example, by column chromatography or high-performance liquid chromatography (HPLC). Preferably, the chromatographic separation is achieved using HPLC on C8, C18 or phenyl-hexyl reversed phase. The mobile phase used for chromatographic purification may contain water and 3-40 vol. % of methanol, ethanol or acetonitrile. Optionally, 0.01 - 0.1 mol/L of a buffer is used in the mobile phase, wherein the buffer comprises ammonium formate pH = 7.0 or ammonium acetate pH = 7.0. Fractions containing the desired metal chelate are collected and evaporated or lyophilized.

In one embodiment, step c) of radiolabelling the Gd(III) chelate from step b) with ¹⁸F is performed by using either ¹⁹F to ¹⁸F isotopic exchange on the pyridine moiety of the Gd(III) chelate, wherein R is F; or by using nucleophilic aromatic substitution of nitro group with ¹⁸F on the pyridine moiety of the Gd(III) chelate, wherein R is nitro group.

Preferably, step c) is performed under the following conditions:
The Gd(III) chelate from step b) is dissolved in a solvent, preferably selected from the group comprising acetone, acetonitrile, dichloromethane, N,N-dimethylformamide (DMF), N,N-dimethylacetamide (DMAC), dimethylpropyleneurea (DMPU), dimethylsulfoxide (DMSO), sulfolane, ethyl acetate, hexamethylphosphoramide (HMPA), pyridine, tetrahydrofuran (THF), methanol, ethanol, propanol, isopropanol, butanol, tert-butanol, N-methyl-2-pyrrolidone (NMP), 1,3-dimethyl-2-imidazolidinone, *tert-*amyl alcohol, water or a mixture thereof. Preferably, the final concentration of Gd(III) chelate in the solvent or solvent mixture is in the range of from 10⁻¹² M to 2 M. Optionally, a source of non-radioactive F⁻ ions is added to achieve a concentration in the range of from 10⁻¹² M to 2 M. The source of non-radioactive F⁻ ions may be, for example: tetramethylammonium fluoride, tetraethylammonium fluoride, tetrapropylammonium fluoride, tetrabutylammonium fluoride, tetrahexylammonium fluoride, NaF, KF, RbF, CsF, [Na(15-crown-5)⁺]F⁻, [K(18-crown-6)⁺]F⁻, [Cs(21-crown-7)⁺]F⁻, [Na(cryptand[2.2.1])⁺]F⁻, [K(cryptand[2.2.2])⁺]F⁻ or [Cs(cryptand[322])⁺]F⁻. The source of non-radioactive F⁻ ions is added in order to achieve a nucleophilic aromatic substitution of NO₂ group with ¹⁹F. If the Gd(III) chelate from step b) does not contain any NOz group, then addition of non-radioactive F⁻ ions can be omitted.

Analogously, source of radioactive ¹⁸F⁻ ions is added, in the amount that corresponds to the desired activity. In terms of molar equivalents, this represents only a fraction of the amount of the Gd(III) chelate and/or of the non-radioactive F⁻ ions. The amount (activity) of ¹⁸F⁻ ions would be known to a person skilled in the art without exerting any inventive activity. The source of radioactive ¹⁸F⁻ ions is analogous to the sources of non-radioactive F⁻ ions listed above (e.g. [¹⁸F]tetramethylammonium fluoride, [¹⁸F]tetraethylammonium fluoride, [¹⁸F]tetrapropylammonium fluoride, [¹⁸F]tetrabutylammonium fluoride, [¹⁸F]tetrahexylammonium fluoride, Na¹⁸F, K¹⁸F, Rb¹⁸F, Cs¹⁸F, [Na(15-crown-5)+]¹⁸F⁻, [K(18-crown-6)⁺]¹⁸F⁻, [Cs(21-crown-7)⁺]¹⁸F⁻, [Na(cryptand[2.2.1])⁺]¹⁸F⁻, [K(cryptand[22.2])⁺]¹⁸F⁻ or [Cs(cryptand[3.22])⁺]¹⁸F⁻). The reaction mixture is then stirred at a temperature in the range of from -50 °C to 250 °C, preferably in the range of from 5 °C to 200 °C, for at least 0.1 s, preferably for at most 8 hours.

In a more preferred embodiment, step c) is performed in the solvent selected from acetonitrile, DMF, DMAC, DMPU, DMSO, sulfolane, HMPA, THF, *tert*-butanol, NMP, 1,3-Dimethyl-2-imidazolidinone, *tert*-amyl alcohol, water or a mixture thereof. The final concentration of Gd(III) chelate in the solvent or solvent mixture is in the range of from 10⁻⁶ M to 0.5 M. Optionally, a source of non-radioactive F⁻ ions is added to achieve a concentration in the range of from 10⁻⁶ M to 0.5 M. The source of non-radioactive F⁻ions is preferably selected from: tetramethylammonium fluoride, tetrabutylammonium fluoride, NaF, KF, CsF, [Na(15-crown-5)⁺]F⁻, [K(18-crown-6)⁺]F⁻, [Na(cryptand[2.2.1])⁺]F⁻ and [K(cryptand[2.2.2])⁺]F⁻. A source of radioactive ¹⁸F⁻ ions is added, in the amount that corresponds to the desired activity. The source of radioactive ¹⁸F⁻ ions is analogous to the sources of non-radioactive F⁻ ions listed above (e.g. [¹⁸F]tetramethylammonium fluoride, [¹⁸F]tetrabutylammonium fluoride, Na¹⁸F, K¹⁸F, Cs¹⁸F, [Na(15-crown-5)⁺]¹⁸F⁻, [K(18-crown-6)⁺]¹⁸F⁻, [Na(cryptand[2.2.1])⁺]¹⁸F⁻ or [K(cryptand[2.2.2])⁺]¹⁸F⁻. The reaction mixture is then stirred at a temperature in the range of from 25 °C to 200 °C for at least 1 minute, preferably for at most 2 hours.

Most preferably, the solvent is selected from acetonitrile, DMF, DMSO or mixtures thereof. The final concentration of Gd(III) chelate is in the range of from 0.1 mM to 100 mM. Optionally, a source of non-radioactive F⁻ ions is added to achieve a concentration in the range of from 1 mM to 300 mM. The source of non-radioactive F⁻ ions is preferably selected from tetrabutylammonium fluoride or [K(cryptand[2.2.2])⁺]F⁻. A source of radioactive ¹⁸F⁻ ions is added, in the amount that corresponds to the desired activity. The source of radioactive ¹⁸F⁻ is selected from [¹⁸F]tetrabutylammonium fluoride and [K(cryptand[22.2])⁺]¹⁸F⁻. The reaction mixture is then stirred at a temperature in the range of from 25 °C to 150 °C for at least 1 minute, preferably for at most 100 minutes.

The object of the present invention is also a method of imaging a region of interest in a patient using the multimodal PET/MRI imaging, the method comprising:
- administering to a patient a pharmaceutical formulation comprising the multimodal PET/MRI contrast agent of general formula (VI), (VIa) or (VIb);
- detecting arrival of the multimodal PET/MRI contrast agent present in the formulation in the region of interest by comparing the PET and/or MRI signals in said region of interest during or after administering said pharmaceutical formulation;
- collecting PET and MRI image data of the region of interest; and
- constructing a multimodal PET/MRI image of said region of interest using the PET and MRI image data, wherein the region of interest appears distinct from the background tissue.

The pharmaceutical formulation comprising the multimodal PET/MRI contrast agent of general formula (VI), (VIa) or (VIb) may further contain conventionally used pharmaceutically acceptable auxiliary substances. The dosage form of the pharmaceutical preparation is a form for administration by injection, most often as a bolus or as an infusion, preferably intravenously. Suitable pharmaceutically acceptable auxiliary substances are preferably selected from the group containing solvents (especially aqueous or saline solution), buffers (especially phosphate buffer, HEPES = 2-[4-(2-hydroxyethyl)piperazine-1-yl]ethanesulfonic acid), ionization additives, antioxidants, antimicrobial additives. A person skilled in the art would be able, without exerting inventive activity, to determine which auxiliary substance to choose.

In one embodiment, the pharmaceutical formulation further contains a coordination compound of Gd(III) ion and the cyclen based compound of general formula (I) or (Ia) as defined above. The presence of said coordination compound, containing Gd(III) and ¹⁹F, provides for the MRI signal, while the presence of the multimodal PET/MRI contrast agent of general formula (VI), (VIa) or (VIb), bearing ¹⁸F, provides for the PET and MRI signal. As the amount of ¹⁸F radionuclide needed for the PET signal is about 9-10 orders of magnitude lower than the amount of Gd(III) chelate needed for the MRI contrast, MRI contrast of the pharmaceutical formulation may be enhanced by the presence of non-radioactive Gd(III) chelate of the compound of general formula (I) or (Ia) as defined above. It is preferred if the radioactive and non-radioactive Gd(III) chelates differ only in the fluorine isotope (meaning that the coordination compounds are structurally identical, differing only in whether bearing ¹⁹F or ¹⁸F isotope). The concentrations of the non-radioactive Gd(III) chelate should be sufficient to achieve MRI contrast, the concentration of the PET/MRI contrast agent should be sufficient to achieve PET contrast. The concentrations of Gd(III) chelate to achieve MRI contrast, and of the ¹⁸F radionuclide to achieve the PET contrast, would be known to the skilled person. In general, the molar ratio of non-radioactive Gd(III) chelate of compound of general formula (I) or (Ia) to the PET/MRI contrast agent of general formula (VI), (VIa) or (VIb) should preferably be from about 1:10⁻¹⁰ to about 1:10⁻⁸.

The object of the present invention is also the multimodal PET/MRI contrast agent of general formula (VI), (VIa) or (VIb) and/or the pharmaceutical formulation containing thereof, for use in medicine, preferably in combined PET/MRI diagnostic methods.

### Brief description of Drawings

**Figure 1****:** Chromatograms from radiolabelling experiment from Example 4 with [**Gd(L¹)**] as precursor. Product corresponds to peak at 7.431 minutes in UV detector. In gamma detector, product corresponds to peak at 7.767 minutes.
**Figure 2****:** Chromatograms from radiolabelling experiment from Example 4 with **[Gd(L⁴)]** as precursor. Product corresponds to peak at 7.343 minutes in UV detector. In gamma detector, product corresponds to peak at 7.700 minutes.
**Figure 3****:** Chromatograms from radiolabelling experiment from Example 4 with **L⁴** as a precursor. It can be seen that no radiolabelled product is present, the only peak in gamma detector corresponds to the free ¹⁸F⁻. The main peak in UV detector corresponds to unlabelled starting material **L⁴**.
**Figure 4****:** Determination of relaxivities of **[Gd(L⁴)]** and **[Gd(L⁷)]** by linear regression of their concentration versus longitudinal relaxation rate at 37 °C and 0.47 T.

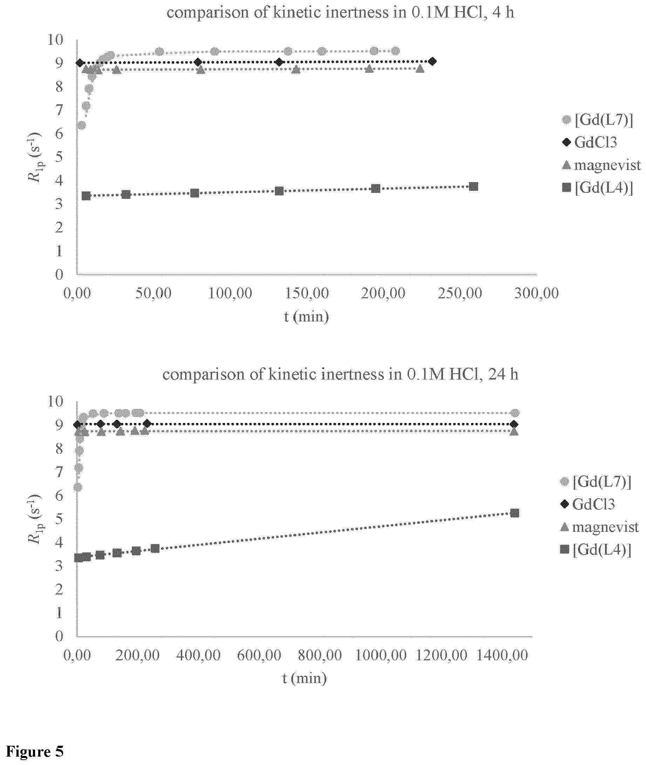

**Figure 5****:** Comparison of kinetic inertness of **[Gd(L⁴)]**, **[Gd(L⁷)]** and Magnevist by acid-assisted decomplexation in 0.1M HCl at 37 °C by measurement of relaxation rate on a relaxometer. The top graph shows data taken during the first 4 hours of the experiment and the bottom graph adds a data point after 24 hours. The *R*₁ₚ value should be the same for all measured complexes after reaching thermodynamic equilibrium but elemental analysis confirmed minor differences in concentrations of the samples (pipetting error) that explain the difference. Data for unchelated GdCl₃ solution of the same molar concentration is shown for reference. The curves demonstrate the differences in kinetic inertness of the metal chelates. While clinically used Magnevist is decomposed almost immediately (< 5 min), **[Gd(L⁷)]** takes longer time to reach complete decomplexation. Finally, the **[Gd(L⁴)]** chelate is the most inert and even after 24 hours under these conditions the curve is far from reaching the thermodynamic equilibrium, demonstrating that most of the chelate remains intact.

### Examples

### Example 1: synthesis of intermediates

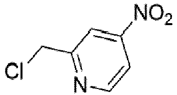

**Synthesis of intermediate (1):** In a 250 mL round bottom glass flask, **2-methyl-4-nitropyridine *N*-oxide** (5.15 g, 33.4 mmol, 1.0 equiv.) was dissolved in DCM (200 mL). Solution of **TFAA** (14mL, 101 mmol, 3.0 equiv.) in DCM (35 mL) was added dropwise and the resulting suspension was stirred for 96 h at RT. Saturated aqueous solution of K₂CO₃ (40 mL) was added in four portions over period of 1 h and reaction mixture was stirred until bubbling stopped. Water phase was extracted with DCM (2×200 mL) and solvent was evaporated on rotary evaporator. The residue was purified by preparative HPLC (C18, MeCN/H₂O gradient with 0.1 % FA in the mobile phase). Fractions with product were joined and lyophilized. The resulting white solid was dissolved in DCM (340 mL) followed by addition of **DMF** (46 µL, 0.60 mmol, 0.02 eqiv.) and **SOCl₂** (5.5 mL, 75.1 mmol, 2.25 eqiv.) dropwise. After 6 hours of stirring at RT the reaction mixture was put on ice bath and was quenched by careful addition of saturated aqueous solution of NaHCO₃ (200 mL). Phases were separated and water phase was extracted with DCM (200 mL). Organic phase was dried over Na₂SO₄, filtered and liquids were evaporated on rotary evaporator. The residue was purified by column chromatography (SiOz, 100 % DCM to 10% MeOH in DCM). Fractions containing product were pooled and evaporated to dryness to yield the product as yellow oil. **Yield:** 2.2 g (38%; 2 steps; based on **2-methyl-4-nitropyridine N-oxide). NMR** (**CDCl₃**): **¹H** δ_{H} 4.80 (C*H*₂-arom, s, 2H); 7.98 *(arom,* dd, ³*J*_{HH}=5, ⁴*J*_{HH}= 2, 1H); 8.24 *(arom,* d, ⁴*J*_{HH}=2, 1H); 8.87 *(arom,* d, ³*J*_{HH}=5, 1H); **¹³C{¹H}** δ_{C} 45.8 (*C*H₂-arom, s); 115.6 *(arom,* s); 115.8 *(arom,* s); 151.8 *(arom,* s); 154.8 *(arom,* s); 160.2 *(arom,* **s).EI-HRMS:** 172.0033 [M]⁺ (theor. [C₆H₅O₂N₂Cl₁]⁺ = 172.0034).

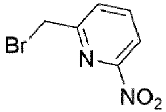

**Synthesis of intermediate (2):** In a 50 mL pear-shaped glass flask, **2-methyl-6-nitropyridine** (224 mg, 1.62 mmol, 1.0 equiv.), **NBS** (577 mg, 3.24 mmol, 2.0 equiv.) and **AIBN** (42 mg, 0.26 mmol, 0.16 equiv.) were dissolved in CCl₄ (6 mL). The reaction mixture was heated under reflux for 19 hours.The solvent was evaporated on rotary evaporator. The residue was purified by preparative HPLC (C18, MeCN/H₂O gradient with 0.1 % FA in the mobile phase). Fractions containing the product were pooled and DCM was added until separation of phases occurred. Organic phase was separated and washed with saturated aqueous NaHCO₃. Organic phase was concentrated on rotary evaporator,giving product as colourless solid. **Yield:** 79 mg (23 %, 1 step, based on **2-methyl-6-nitropyridine). NMR** (CDCl₃): **¹H** δ_{H} 4.63 (C*H*₂-arom, s, 2H); 7.87 *(arom,* dd, ²*J*_{HH}=7.6,³*J*_{HH}= 0.9, 1H); 8.07 *(arom,* t, ²*J*_{HH}=7.8, 1H); 8.19 *(arom,* dd, ²*J*_{HH}=8.0, ³*J*_{HH}= 0.8, 1H); **¹³C{¹H}** δ_{C} 31.7 (*C*H₂-arom, s); 117.3 *(arom,* s); 129.3 *(arom,* s); 141.1 *(arom,* s); 156.2 *(arom,* s); 157.5 *(arom,* **s).CI-HRMS:** 216.9605 [M+H]⁺(theor. [C₆H₆O₂Br₁N₂]⁺ = 216.9607).

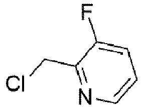

**Synthesis of intermediate (3):** In a 50 mL round bottom glass flask, **3-fluoropicolinic acid** (813 mg, 5.76 mmol, 1.0 equiv.) was dissolved in MeOH (12 mL) and **H₂SO₄** (300 µL, 5.40 mmol, 0.94 eqiv.) was added dropwise. The reaction mixture was heated under reflux for 41 hours. The reaction mixture was cooled to RT and quenched with saturated aqueous NaHCO₃ (1 mL). Reaction mixture was concentrated to approximately 2 ml on rotary evaporator and was let stand until crystals started to appear. Crystals were washed with H₂O (2 × 2 mL) and dried under vacuum. The resulting crystals were dissolved in MeOH (30 mL) and **NaBH₄** (370 mg, 9.78 mmol, 1.70 eqiv.) was added in three portions while stirring on ice bath. After 30 minutes reaction mixture was allowed to reach RT and continued stirring for another 1.5 hours. Reaction was quenched with H₂O (1 mL) and liquids were evaporated on rotary evaporator. Residue was suspended in saturated aqueous K₂CO₃ (50 mL) and extracted with DCM (2×50 mL). The solvent was evaporated on rotary evaporator and residue was dissolved in DCM (7 mL) followed by addition of **DMF** (5 µL, 0.06 mmol, 0.01 eqiv.) and **SOCl₂** (0.523 mL, 1.17 mmol, 0.20 eqiv.) was added dropwise. After 1 hour of stirring at RT the reaction mixture was quenched by careful addition of saturated aqueous solution of NaHCO₃ (10 mL). Phases were separated and water phase was extracted with DCM (10 mL). Organic phase was dried over Na₂SO₄, filtered and liquids were evaporated on rotary evaporator to yield the product as yellowish oil. **Yield:** 186 mg (22%; 3 steps; based on **3-fluoropicolinic acid). NMR** (CDCl₃): **¹H** δ_{H} 4.68 (C*H*₂-arom, s, 2H); 7.19-7.29 (*arom.,* m, 1H); 7.32-7.42 *(arom.,* m, 1H); 8.31-8.40 *(arom.,* m, 1H); **¹³C{¹H}** δ_{C} 40.6 (*C*H₂-arom, s); 123.9 *(arom,* d, ²*J*_{CF} = 18.6 Hz); 125.4 *(arom,* d, ³*J*_{CF} = 4.0 Hz); 144.9 *(arom,* d, ²*J*_{CF} = 14.2 Hz); 145.5 *(arom,* d, ⁴*J*_{CF}= 5.4 Hz); 157.8 *(arom,* d, ¹*J*_{CF} = 260.8 Hz). **¹⁹F{¹H}** *δ*_{F} -123.2 (s). **CI-HRMS:** 146.0174 [M+H]⁺(theor. [C₆H₆Cl₁N₁F_{1]}⁺ = 146.0167).

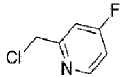

**Synthesis of intermediate (4):** In a 50 mL round bottom glass flask, **(*4*-fluoropyridin-2-yl)methanol** (227 mg, 1.79 mmol, 1.0 equiv.) was dissolved in DCM (35 mL) and DMF (4 µL, 0.05 mmol, 0.01 equiv.) was added. The reaction mixture was put on ice bath. After 20 minutes on ice bath, thionyl chloride (400 µL, 5.36 mmol, 3.0 equiv.) was added dropwise and reaction mixture was let stir on ice bath. After 15 minutes, the reaction mixture was allowed to let warm to RT. After 1 hour of stirring at RT the reaction mixture was put on ice bath and quenched by careful addition of saturated aqueous solution of NaHCO₃ (10 mL). Phases were separated, and the water phase was extracted with DCM (2 × 40 mL). Organic phase was dried over Na₂SO₄, filtered and liquids were evaporated on rotary evaporator to yield the product as colorless oil. **Yield:** 217 mg (83%; 1 step; based on **(4-fluoropyridin-2-yl)methanol) NMR** (CDCl₃): **¹H** (400.1 MHz, *T* = 300 K) *δ*_{H} 4.69 (C*H*₂-arom, s, 2H); 7.00 *(arom.,* ddd, *J=* 8, 6, 2 Hz 1H); 7.24-7.30 *(arom.,* m, 1H); 8.55 *(arom.,* dd, *J=* 8, 6 Hz H). **¹³C{¹H}** (100.6 MHz, *T=* 300 K) *δ*_{C} 70.8 (*C*H₂-arom, d, ⁴*J*_{CF} = 3 Hz); 110.9 *(arom,* d, ²*J*_{CF} = 18 Hz); 111.2 *(arom,* d, ²*J*_{CF} = 17 Hz); 151.8 *(arom,* d, ³*J*_{CF} = 7 Hz); 160.0 *(arom,* d, ³*J*_{CF} = 7 Hz); 169.4 *(arom,* d, ¹*J*_{CF} = 263 Hz). **¹⁹F{¹H}** (376.5 MHz, *T* = 300.0 K) *δ*_{F} - 100.9 (s). **ESI-MS:** 146.0 [M+H]⁺(theor. [C₆H₆N₁F₁Cl₁]⁺ = 146.0).

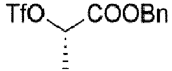

**Synthesis of intermediate (5):** Pear-shaped glass flask (100 mL) was charged with **benzyl l-lactate** (900 mg; 4.99 mmol; 1.00 equiv.) and magnetic stirrer and three times briefly secured with argon. Under constant flow of argon was then added dry DCM (20 mL) through septum and the mixture was cooled with an ice bath (5 °C) followed by dropwise addition of **triflic anhydride** (0.88 mL; 5.23 mmol; 1.05 equiv.) and immediately followed by dropwise addition of dry **pyridine** (0.42 ml; 5.25 mmol; 1.05 equiv.). The resulting mixture was stirred at 5 °C for 30 min. Resulting suspension was then directly purified by column chromatography (30 g SiOz, DCM). Combined fractions with product were evaporated to dryness and briefly dried on high vacuum to give product in the form of free base as nearly colourless oil. **Yield:** 1.12 g (77%; 1 step; based on **benzyl l-lactate**). **NMR (DMSO-d*₆*)**: **¹H** δ_{H} 1.53 (C*H*₃, d, 3H, ³*J*_{HH} = 7); 5.27 (C*H*₂, s, 2H); 5.34 *(CH,* q, 1H, ³*J*_{HH} = 7); 7.17-7.65 *(Ph,* m, 5H). **¹⁹F{¹H}** δ_{F} -77.7 (s). **ESI-HRMS:** 335.0175 [M+Na]⁺ (theor. [C₁₁H₁₁O₅F₃S₁Na₁]⁺ = 335.0172).

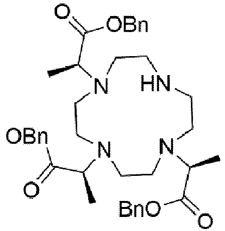

**Synthesis** of **intermediate (6):** In a glass vial (20 mL), **cyclen** (free base; 100 mg; 580 µmol; 1.0 equiv.) and **K₂CO₃** (160 mg; 1.16 mmol; 2.0 equiv.) were suspended in dry MeCN (6 mL) followed by addition of **5** (362 mg; 1.16 mmol; 2.0 equiv.) in dry MeCN (4 mL). The resulting mixture was stirred at 40°C for 1 h. Solids were filtered off using syringe microfilter (PTFE) and filtrate was evaporated to dryness. Residue was purified by preparative HPLC (C18; H₂O-MeCN gradient with TFA additive). Fractions with product were joined and lyophilized to give product in the form of TFA salt as brown honey. **Yield:** 106 mg (25 %, 1 step, based on cyclen). **NMR** (CD₃CN): **¹H** δ_{H} 120 (C*H*₃, d, 3H, ³*J*_{HH} = 7 Hz); 1.26 (C*H*₃, d, 6H, ³*J*_{HH} = 7 Hz); 2.30-2.96 *(mc,* m, 16H); 3.59 (C*H*CH₃-COOBn, q, 2H, ³*J*_{HH} = 7 Hz); 3.77 (C*H*CH₃-COOBn, q, 1H, ³*J*_{HH} = 7 Hz); 5.09 (C*H*₂-arom, s, 2H); 5.10 (C*H*₂-arom, s, 4H); 7.29-7.41 *(arom,* m, 15H). **ESI-HRMS:** 659.3797 [M+H]⁺ (theor. [C₃₈H₅₁N₄O₆]⁺ = 659.3803). **EA** (C₃₈H₅₀N₄O₆·0.3TFA·2.0H₂O, *M*_{R} = 729.0): C 63.6 (63.0); H 7.5 (6.9); N 7.7 (7.4); F 2.4 (2.2).

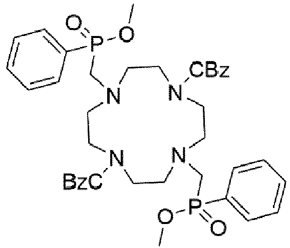

**Synthesis of intermediate** (7): In a glass vial (20 mL), **Cbz2cyclen** (free base; 242 mg; 549 µmol; 1.0 equiv.) was dissolved in MeCN (12 mL) followed by addition of solid (**CH₂O**)***ₙ*** (50 mg; 1.67 mmol; 3.0 equiv.) and **PhP(OMe)₂** (350 µL; 2.2 mmol; 4.0 equiv.). The resulting suspension was stirred 24 h at 80°C. Reaction mixture was then filtered through syringe microfilter (PTFE) and the filtrate was evaporated to dryness. Residue was purified by preparative HPLC (C18; H₂O-MeCN gradient with TFA additive). Fractions with product were combined, neutralized with dil. aq. NaHCO₃ and evaporated to dryness. Residue was dissolved in DCM (75 mL) and H₂O (75 mL) and transferred to a separatory funnel. After shaking, the bottom phase was separated. Aqueous phase was further extracted with DCM (3 × 25 mL). Combined organic layers were dried with anhydrous Na₂SO₄, filtered through glass frit (S3) and evaporated to dryness. The residue was further dried on high vacuum overnight to give product in the form of free base as nearly colourless oil. **Yield:** 259 mg (61%; 1 step; based on **Cbz2cyclen). NMR (CD₃CN): ¹H** δ_{H} 2.30-3.38 (*mc,* C*H*₂-P, m, 16+4H); 3.47 (C*H*₃, d, 6H, ³*J*_{HP} = 11); 5.00-5.12 (C*H*₂-Ph, m, 4H); 7.27-7.40 *(Ph,* m, 10H); 7.40-7.51 *(Ph,* m, 4H); 7.51-7.60 *(Ph,* m, 2H); 7.60-7.82 *(Ph,* m, 4H). **³¹P** δ_{P} 42.8 (m). **ESI-HRMS:** 777.3178 [M+H]⁺ (theor. [C₄₀H₅₁N₄O₈P_{2]}⁺ = 777.3177).

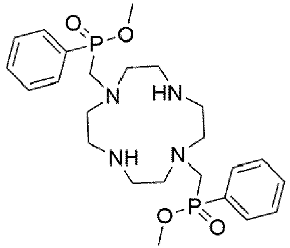

**Synthesis of intermediate (8):** Pear-shaped glass flask (50 mL) was charged with 7 (250 mg; 322 µmol and magnetic stirrer and three times secured with argon. Solid **Pd@C** (50 mg) was then added followed by another securing with argon (three times). Under constant flow of argon was then added MeOH (25 mL) through septum. Argon input was then removed and **Hz gas** (from balloon) was allowed to bubble through the mixture for 2 h at RT. Catalyst was then filtered off using syringe microfilter (PTFE; filter was further washed with MeOH). Filtrate was evaporated to dryness and once co-evaporated with DCM. The residue was further dried on high vacuum overnight to give product in the form of free base as nearly colourless oil. **Yield:** 161 mg (98%; 1 step; based on **8). NMR (CD₃CN): ¹H** δ_{H} 2.28-3.14 (*mc,* C*H*₂-P, m, 16+4H); 3.53 (C*H*₃, d, 3H, ³*J*_{HP} = 11); 3.54 (C*H*₃, d, 3H, ³*J*_{HP} = 11); 7.46-7.63 *(Ph,* m, 6H); 7.71-7.83 *(Ph,* m, 4H). **³¹P** δ_{P} 43.3 (m). **ESI-HRMS:** 509.2438 [M+H]⁺ (theor. [C₂₄H₃₉N₄O₄P₂]⁺ = 509.2441).

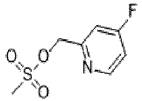

**Synthesis of intermediate (9):** In a 50 mL round bottom glass flask, **methyl *4-*fluoropicolinate** (250 mg, 1.61 mmol, 1.0 equiv.) was dissolved in MeOH (16 mL) and **NaBH₄** (180 mg, 4.78 mmol, ~3.0 equiv.) was added while stirring. The reaction was stirring for 3 hours at RT after which H₂O (1 mL) was added. Liquids were evaporated on rotary evaporator and residue was suspended in saturated aqueous solution of K₂CO₃ (25 mL). Water phase was extracted with DCM (2×25 mL) and solvent was evaporated on rotary evaporator. The residue was dissolved in DCM (11 mL) and **Et₃N** (280 µL, 2.02 mmol, 1.3 eqiv.) was added. The reaction mixture was put on ice bath. After 20 minutes on ice bath **methanesulfonyl chloride** (146 µL, 1.88 mmol, 1.23 eqiv.) was added and reaction mixture was stirred on ice bath. After 15 minutes the reaction mixture was let to warm to RT a was let stirring for 1 hour. Reaction mixture was diluted with DCM (40 mL) and washed with H₂O (2×40 mL), brine (40 mL). Organic phase was dried over Na₂SO₄, filtered and liquids were evaporated on rotary evaporator to yield the product as dark orange oil. **Yield:** 240 mg (77%; 2 steps; based on **methyl 4-fluoropicolinate) NMR** (CDCl₃): **¹H** δ_{H} 3.12 (C*H*₃, s, 3H); 5.33 (C*H*₂-arom, s, 2H); 7.04 (*arom*.,ddd, *J* = 8, 6, 3 Hz 1H); 7.24 *(arom.,* dd, *J=* 9, 3 Hz, 1H); 8.58 *(arom.,* dd, *J=* 8, 6 Hz, 1H); **¹³C{¹H}** δ_{C} 38.2 (*C*H₃, s); 70.4 (*C*H₂-arom, s); 110.5 *(arom,* d, ²*J*_{CF} = 18 Hz); 111.7 *(arom,* d, ²*J*_{CF} = 17 Hz); 152 *(arom,* d, ³*J*_{CF} = 7 Hz); 157.2 *(arom,* d, ³*J*_{CF} = 7 Hz); 169.5 *(arom, d,* ¹*J*_{CF} = 265 Hz). **¹⁹F{¹H}** *δ*_{F}-99.3 (s). **CI-HRMS:** 206.0281 [M+H]⁺(theor. [C₇H₉O₃N₁F₁S₁]⁺ = 206.0282).

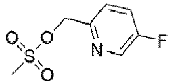

**Synthesis of intermediate (10):** In a 50 mL round bottom glass flask, **(5-fluoropyridin-2-yl)methanol** (173 mg, 1.36 mmol, 1.0 equiv.) was dissolved in DCM (10 mL) and **Et₃N** (250 µL, 1.80 mmol, 1.3 eqiv.) was added. The reaction mixture was put on ice bath. After 20 minutes on ice bath **methanesulfonyl chloride** (142 µL, 1.84 mmol, 1.35 eqiv.) was added and reaction mixture was stirred on ice bath. After 15 minutes the reaction mixture was let warm to RT a was let stirring for another 1 hour. Reaction mixture was diluted with DCM (40 mL) and washed with H₂O (2×40 mL), brine (40 mL). Organic phase was dried over Na₂SO₄, filtered and liquids were evaporated on rotary evaporator to yield the product as orange solid. **Yield:** 219 mg (78%; 1 steps; based on **(5-fluoropyridin-2-yl)methanol) NMR** (CDCl₃): **¹H** δ_{H} 3.08 (C*H*₃, s, 3H); 5.31 (C*H*₂-arom, s, 2H); 7.42 - 7.53 (*arom*.,m, 2H); 8.47 *(arom.,* dd, *J*= 3, 1 Hz, 1H); **¹³C{¹H}** δ_{C} 38.2 (*C*H₃, s); 70.8 (*C*H₂-arom, s); 124.1 *(arom,* d, ³*J*_{CF} = 3 Hz); 124.2 *(arom,* d, ²*J*_{CF} = 11 Hz); 138 *(arom,* d, ²*J*_{CF} = 24 Hz); 149.6 *(arom,* d, ⁴*J*_{CF} = 4 Hz); 159.4 *(arom,* d, ¹*J*_{CF} = 258 Hz). **¹⁹F{¹H}** *δ*_{F} - 125.8 (s). **CI-HRMS:** 206.0284 [M+H]⁺(theor. [C₇H₉O₃N₁F₁S₁]⁺ = 206.0282).

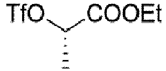

**Synthesis of intermediate (11):** Pear-shaped glass flask (500 mL) was charged with **ethyl _{L}-lactate** (6.70 g; 56.7 mmol; 1.00 equiv.) and magnetic stirrer and three times briefly secured with argon. Under constant flow of argon was then added dry DCM (200 mL) through septum and the mixture was cooled with an ice bath (5 °C) followed by dropwise addition of **triflic anhydride** (10.0 mL; 59.4 mmol; 1.05 equiv.) and immediately followed by dropwise addition of dry **pyridine** (4.80 ml; 59.6 mmol; 1.05 equiv.). The ice bath was then removed and the mixture was further stirred at RT for 1 h. Resulting suspension was then directly purified by column chromatography (250 g SiOz, DCM). Combined fractions with product were evaporated to dryness and briefly dried on high vacuum to give product as faint brown oil. **Yield:** 12.02 g (85%; 1 step; based on ethyl **_{L}-lactate**). **NMR (DMSO-d*₆*)**: **¹H** δ_{H} 1.26 (C*H*₃-CH₂, t, 3H, ³*J*_{HH} = 7); 1.51 (C*H*₃-CH, d, 3H, ³*J*_{HH} = 7); 4.19-4.31 (C*H*₂, m, 2H); 5.27 (C*H*, q, 1H, ³*J*_{HH} = 7). **¹⁹F{¹H}** δ_{F} -77.7 (s). **CI-HRMS:** 251.0193 [M+H]⁺ (theor. [C₆H₁₀O₅S₁F₃]⁺ = 251.0196).

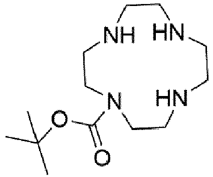

**Synthesis of intermediate (12):** In a pear shaped glass flask (25 mL), cyclen (free base; 200 mg; 1160 µmol; 1.0 equiv.) was dissolved in dry DCM (12 mL) followed by dropwise addition **of *tert*-butyl 4-nitrophenyl carbonate** (278 mg; 1.16 mmol; 1.0 equiv.) in dry DCM (4 mL). The resulting mixture was stirred at RT for 30 minutes. Liquids were evaporated and residue was purified by preparative HPLC (C18; H₂O-MeCN gradient with TFA additive). Fractions with product were joined and lyophilized to give product in the form of TFA salt as transparent solid. **Yield:** 245 mg (42 %, 1 step, based on **cyclen** assuming **12**·2TFA). **NMR (CD₃CN): ¹H** δ_{H} 1.44 (C*H*₃, s, 9H); 3.01-3.08 (*mc*, m, 4H); 3.11-3.18 (*mc*, m, 4H); 3.23-3.29 *(mc,* m, 4H); 3.39-3.48 *(mc,* m, 4H). **¹³C{¹H}** (100.6 MHz, *T=* 300 K) δC 27.48 (*C*H₃, s); 44.14 *(mc,* s); 44.70 *(mc,* s); 47.69 *(mc,* s); 47.96 *(mc,* s); 80.57 (*C*-CH₃, s); 155.66 (*C*O, s). **ESI-HRMS:** 273.2284 [M+H]⁺ (theor. [C₁₃H₂₉N₄O₂]⁺ = 273.2285).

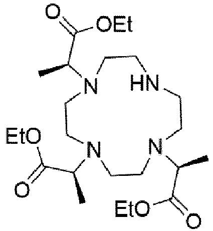

**Synthesis of intermediate (13):** In a glass vial (20 mL), **intermediate 12** (100 mg; 200 µmol; 1.0 equiv.) and **11** (367 mg; 1.47 mmol; 7.4 equiv.) were dissolved in dry MeCN (15 mL) followed by addition of **K₂CO₃** (254 mg; 1.84 mmol; 9.2 equiv.). The resulting mixture was stirred at RT for 15 h. Solids were filtered off using syringe microfilter (PTFE) and filtrate was evaporated to dryness. Residue was purified by preparative HPLC (C18; H₂O-MeCN gradient with FA additive). Fractions containing pure product in the form of ethyl ester were pooled, evaporated to dryness. The residue was dissolved in neat **TFA** (3 mL) and stirred for 15 h at RT. Volatiles were removed on rotary evaporator and the residue was purified on preparative HPLC (C18, MeCN/H₂O gradient with 0.1 % FA in the mobile phase). Fractions with product were joined and lyophilized to give product as transparent honey. **Yield:** 21 mg (20 %, 2 steps, based on 12 assuming **13**·FA). **NMR (DMSO-d*₆*)**: **¹H** δ_{H} 1.17-1.24 (C*H*₃, m, 18H); 2.40 (*mc*, m, 2H); 2.55 (*mc*, m, 2H); 2.73-2.94 (*mc*, m, 10H); 3.21 (*mc*, m, 2H); 3.63 (C*H*CH₃-COOEt, q, 2H, ³*J*_{HH} = 7 Hz); 3.74 (C*H*CH₃-COOBn, q, 1H, ³*J*_{HH} = 7 Hz); 4.07-4.13 (O-C*H*₂-CH₃, m, 6H); 8.8 (N*H*, bs, 1H). **¹³C{¹H}** δ_{C} 14.1 (*C*H₃, s); 14.22 (*C*H₃, s); 14.24 (*C*H₃, s); 15.2 (*C*H₃, s); 44.8, 45.2, 46.6, 50.0 (*mc*); 54.8 (CCH₃H-COCH₂CH₃, s); 57.8 (CCH₃H-COCH₂CH₃, s); 60.0 (CCH₃H-COCH₂CH₃, s); 60.04 (CCH₃H-COCH₂CH₃, s); 172.5 (*C*O, s); 172.8 (*C*O, s). **ESI-HRMS:** 473.3329 [M+H]⁺ (theor. [C₂₃H₄₅N₄O₆]⁺ = 473.3334).

### Example 2: synthesis of ligands

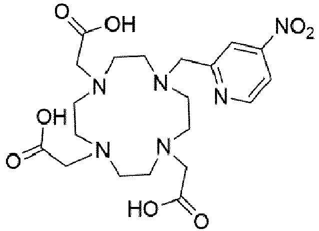

**Synthesis of ligand L¹** (2,2',2"-(10-((4-nitropyridin-2-yl)methyl)-1,4,7,10-tetraazacyclododecane-1,4,7-triyl)triacetic acid): Solution 1 (168 mg, 0.97 mmol, 1.1 equiv.) was added to a solution of ***t*-BuDO3A**·HBr (527 mg, 0.89 mmol, 1.0 equiv.) in MeCN (18 mL) followed by addition of dried **K₂CO₃** (489 mg, 3.54 mmol, 4.0 equiv.) and the resulting suspension was stirred for 24 hours at RT. The solids were filtered off and the filtrate was concentrated on rotary evaporator. Resulting oil was purified on preparative HPLC (C18, MeCN/H₂O gradient with 0.1 % FA in the mobile phase). Fractions containing pure product in the form of *tert*-butyl ester were pooled, evaporated to dryness and several times co-evaporated with MeOH to remove MeCN. The residue was dissolved in neat **TFA** (6 mL) and stirred for 16 h at RT. Volatiles were removed on rotary evaporator and the residue was purified on preparative HPLC (C18, MeCN/H₂O gradient with 0.1 % TFA in the mobile phase). Fractions with product were joined and lyophilized to give product in the form of TFA salt as yellowish solid. **Yield:** 342 mg (56 %, 2 steps, based on ***t*-BuDO3A**·HBr). **NMR** (D₂O): **¹H** δ_{H} 3.09-3.70 (*mc*, C*H*₂-COOH, m, 22H); 4.35 (C*H*₂-arom, bs, 2H); 8.14 *(arom,* dd, 1H, ³*J*_{HH} = 6 Hz, ⁴*J*_{HH} = 2 Hz); 8.40 *(arom,* d, 1H, ³*J*_{HH} = 2 Hz); 8.87 *(arom,* d, 1H, ³*J*_{HH} = 6Hz); **¹³C{¹H}** δ_{C} 49.7 *(mc,* s); 50.6 *(mc,* s); 51.0 *(mc,* s); 54.7 *(mc,* s); 55.8 (*C*H₂-COOH, s); 57.8 (*C*H₂-arom, s); 117.4 *(arom,* s); 118.6 *(arom,* s); 152.0 *(arom,* s); 155.8 *(arom,* s); 173.0 (CO, s); 175.5 (CO, s). **ESI-HRMS:** 483.2195 [M+H]⁺ (theor. [C₂₀H₃₁N₆O₈]⁺ = 483.2198). **EA** (C₂₀H₃₀N₆O₈·1.8TFA·1.0H₂O, *M*_{R} = 687.7): C 41.2 (42.0); H 4.7 (5.2); N 12.2 (12.8); F 14.9 (15.9).

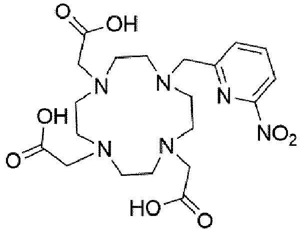

**Synthesis of ligand L²** (2,2',2"-(10-((6-nitropyridin-2-yl)methyl)-1,4,7,10-tetraazacyclododecane-1,4,7-triyl)triacetic acid): Solution 2 (74 mg, 0.34 mmol, 1.1 equiv.) in MeCN (2 mL) was added to a solution of ***t*-BuDO3A**·HBr (185 mg, 0.31 mmol, 1.0 equiv.) in MeCN (42 mL) followed by addition of dried **K₂CO₃** (215 mg, 1.55 mmol, 5 equiv.) and the resulting suspension was stirred for 24 hours at RT. The solids were filtered off and the filtrate was concentrated on rotary evaporator. Resulting oil was purified on preparative HPLC (C18, MeCN/H₂O gradient with 0.1 % FA in the mobile phase). Fractions containing pure product in the form of tert-butyl ester were pooled, evaporated to dryness and several times co-evaporated with MeOH to remove MeCN. The residue was dissolved in neat **TFA** (3 mL) and stirred for 16 h at RT. Volatiles were removed on rotary evaporator and the residue was purified on preparative HPLC (C18, MeCN/H₂O gradient with 0.1 % FA in the mobile phase). Fractions with product were joined and lyophilized to give product in the form of FA salt as yellowish solid. **Yield:** 50 mg (31 %, 2 steps, based on ***t*-BuDO3A**·HBr). **NMR** (D₂O): **¹H** δ_{H} 2.94-3.18 (*mc*, m, 8H); 3.33-3.51 (*mc*, m, 8H); 3.54 (C*H*₂-COOH, s, 2H); 3.61-3.82 (C*H*₂-COOH, m, 4H); 4.05 (C*H*₂-arom, s, 2H); 8.16 *(arom,* t, 1H, ³*J*_{HH} = 8 Hz); 8.24-8.33 *(arom,* m, 2H); **¹³C{¹H}** δ_{C} 48.6 *(mc,* s); 51.1 *(mc,* s); 52.2 *(mc,* s); 54.1 (CH₂-COOH, s); 56.8 (*C*H₂-COOH, s); 58.2 (*C*H₂-arom, s); 118.5 *(arom,* s); 132.2 *(arom,* s); 143.6 *(arom,* s); 155.9 *(arom,* s); 157.8 *(arom,* s); 169.9 (CO, s); 175.3 (CO, s). **ESI-HRMS:** 483.2196 [M+H]⁺ (theor. [C₂₀H₃₂N₅O₆]⁺ = 483.2198). **EA** (C₂₀H₃₀N₆O₈·0.8FA·0.2H₂O, *M*_{R} = 522.9): C 47.8 (47.8); H 62 (6.2); N 16.1 (16.1).

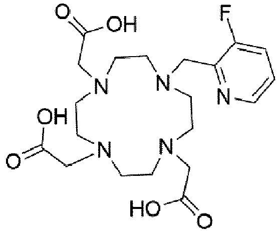

**Synthesis of ligand L³** (2,2',2"-(10-((3-fluoropyridin-2-yl)methyl)-1,4,7,10-tetraazacyclododecane-1,4,7-triyl)triacetic acid): Solution of **3** (106 mg, 0.74 mmol, 1.1 equiv.) in MeCN (1 mL) was added to a solution of ***t*-BuDO3A**·HBr (400 mg, 0.67 mmol, 1.0 equiv.) in MeCN (13.4 mL) followed by addition of dried **K₂CO₃** (464 mg, 3.36 mmol, 5.0 equiv.) and the resulting suspension was stirred for 19 hours at RT. The solids were filtered off and the filtrate was concentrated on rotary evaporator. Resulting oil was purified on preparative HPLC (C18, MeCN/H₂O gradient with 0.1 % FA in the mobile phase). Fractions containing pure product in the form of tert-butyl ester were pooled, evaporated to dryness and several times co-evaporated with MeOH to remove MeCN. The residue was dissolved in neat **TFA** (9 mL) and stirred for 19 h at RT. Volatiles were removed on rotary evaporator and the residue was purified on preparative HPLC (C18, MeCN/HzO gradient with 0.1 % FA in the mobile phase). Fractions with product were joined and lyophilized to give product in the form of FA/TFA salt as white solid. **Yield:** 273 mg (70 %, 2 steps, based on ***t*-BuDO3A**·HBr). **NMR (ref tBuOH)** (D₂O): **¹H** δ_{H} 3.00-3.30 (*mc*, m, 8H); 3.30-3.40 *(mc,* m, 4H); 3.40-3.56 *(mc,* m, 4H); 3.61 (C*H*₂-COOH, s, 2H); 3.68 (C*H*₂-COOH, s, 4H); 4.36 (C*H*₂-arom, s, 2H); 7.74 *(arom,* dt, 1H, ³*J*_{HH} = 9 Hz, ³*J*_{HH} = 5 Hz); 8.00 *(arom,* t, 1H, ³*J*_{HH} = 9 Hz); 8.51-8.57 (*arom,* m, 1H); **¹³C{¹H}** δ_{C} 49.8(*mc*, s); 50.0 (*mc*, s); 50.6 (*mc*, s); 50.9 (*mc*, s); 51.1 (*C*H₂-arom, s); 54.9 (*C*H₂-COOH, s); 55.9 (*C*H₂-COOH, s); 127.5 *(arom,* d, ³*J*_{CF}= 6 Hz); 128.4 (*arom,* d, ²*J*_{CF}= 18 Hz); 140.7 *(arom,* s); 144.6 *(arom,* s); 158.8 *(arom,* d, ¹*J*_{CF}= 255 Hz); 171.3 (*C*O, s); 173.4 (*C*O, s). **¹⁹F{¹H}** *δ*_{F} -122.2 (s). **ESI-HRMS:** 456.2254 [M+H]⁺ (theor. [C₂₀H₃₁N₅O₆F₁]⁺ = 456.2253). **EA** (C₂₀H₃₀N₅O₆F₁·0.7TFA·1.1FA, *M*_{R} = 585.9): C 46.1 (46.4); H 5.7 (5.6); N 12.0 (12.2); F 10.1 (9.7).

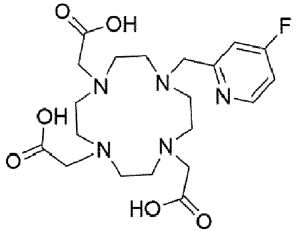

**Synthesis of ligand L⁴** (2,2',2"-(10-((4-fluoropyridin-2-yl)methyl)-1,4,7,10-tetraazacyclododecane-1,4,7 triyl)triacetic acid): Solution of **4** (70 mg, 0.48 mmol, 1.02 equiv.) in MeCN (0.7 mL) was added to a solution of ***t*-BuDO3A**·HBr (281 mg, 0.47 mmol, 1.0 equiv.) in MeCN (9 mL) followed by addition of dried **K₂CO₃** (266 mg, 1.92 mmol, 4 equiv.) and the resulting suspension was stirred for 24 hours at RT. The solids were filtered off and the filtrate was concentrated on rotary evaporator. Resulting oil was purified on preparative HPLC (C18, MeCN/H₂O gradient with 0.1 % FA in the mobile phase). Fractions containing pure product in the form of *tert*-butyl ester were pooled, evaporated to dryness and several times co-evaporated with MeOH to remove MeCN. The residue was dissolved in neat **TFA** (5 mL) and stirred for 16 h at RT. Volatiles were removed on rotary evaporator and the residue was purified on preparative HPLC (C18, MeCN/H₂O gradient with 0.1 % TFA in the mobile phase). Fractions with product were joined and lyophilized to give product in the form of TFA salt as white solid. **Yield:** 208 mg (58 %, 2 steps, based on ***t*-BuDO3A**·HBr). **NMR (ref tBuOH)** (D₂O): ): **¹H** δ_{H} 2.95-3.98 (*mc*, C*H*₂-COOH, m, 22H); 4.20 (C*H*₂-arom, bs, 2H); 7.80 *(arom,* m, 1H); 7.96 *(arom,* m, 1H); 8.85 *(arom,* m, 1H);**¹³C{¹H}** δ_{C} 49.1(*mc*, s); 50.9 (*mc*, s); 52.6 (*mc*, s); 53.6 (*mc*, s); 54.4 (*C*H₂-arom, s); 56.2 (*mc*, s); 115.9 *(arom,* m); 117.7 *(arom,* m); 148.0 *(arom,* m); 155.6 *(arom,* m); 158.8 *(arom,* d, ¹*J*_{CF}= 255 Hz ); 175.4 (CO, s); 176.1 (CO, s). **¹⁹F{¹H}** *δ*_{F} -79.3 (s). **ESI-HRMS:** 456.2250 [M+H]⁺ (theor. [C₂₀H₃₁N₅O₆F₁]⁺ = 456.2253). **EA** (C₂₀H₃₀F₁N₅O₆·2.6TFA·0.3H₂O, *M*_{R} = 757.3): C 40.0 (39.9); H 4.4 (4.5); N 9.3 (9.1); F 22.1 (22.2).

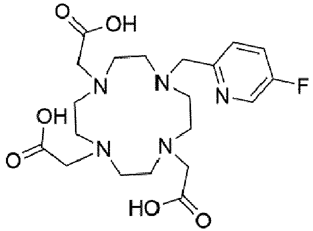

**Synthesis of ligand L⁵** (2,2',2"-(10-((5-fluoropyridin-2-yl)methyl)-1,4,7,10-tetraazacyclododecane-1,4,7-triyl)triacetic acid): Solution of **10** (107 mg, 0.52 mmol, 1.1 equiv.) in MeCN (2 mL) was added to a solution of ***t*-BuDO3A**·HBr (282 mg, 0.47 mmol, 1.0 equiv.) in MeCN (9.5 mL) followed by addition of dried **K₂CO₃** (327 mg, 2.37 mmol, 5.0 equiv.) and the resulting suspension was stirred for 20 hours at RT. The solids were filtered off and the filtrate was concentrated on rotary evaporator. Resulting oil was purified on preparative HPLC (C18, MeCN/H₂O gradient with 0.1 % FA in the mobile phase). Fractions containing pure product in the form of tert-butyl ester were pooled, evaporated to dryness and several times co-evaporated with MeOH to remove MeCN. The residue was dissolved in neat **TFA** (6 mL) and stirred for 19 h at RT. Volatiles were removed on rotary evaporator and the residue was purified on preparative HPLC (C18, MeCN/H₂O gradient with 0.1 % FA in the mobile phase). Fractions with product were joined and lyophilized to give product in the form of FA/TFA salt as white solid. **Yield:** 165 mg (63 %, 2 steps, based on ***t*-BuDO3A**·HBr). **NMR (ref tBuOH)** (D₂O): **¹H** δ_{H} 2.90-3.91 (*mc*, C*H*₂-COOH, m, 22H); 4.30 (C*H*₂-arom, s, 2H); 7.67-8.05 *(arom,* m, 2H); 8.58 *(arom,* s, 1H); **¹³C{¹H}** δ_{C} 50.0, 50.1, 50.2 *(mc);* 50.5 (*C*H₂-COOH, s); 55.3 (*C*H₂-COOH, s); 56.7 (*C*H₂-arom, s); 127.6 *(arom,* s); 128.5 *(arom,* d, ²*J*_{CF}= 17 Hz); 137.1 *(arom,* d, ²*J*_{CF}= 28 Hz); 148.4 *(arom,* s); 160.1 *(arom,* d, ¹*J*_{CF}= 255 Hz ); 171.3 (*C*O, s); 173.4 (*C*O, s). **¹⁹F{¹H}** *δ*_{F} -122.0 (s). **ESI-HRMS:** 456.2254 [M+H]⁺ (theor. [C₂₀H₃₁N₅O₆F₁]⁺ = 456.2253). **EA** (C₂₀H₃₀N₅O₆F₁·0.7TFA·1.3FA, *M*_{R} = 595.1): C 45.8 (45.6); H 5.6 (5.6); N 11.8 (11.7); F 9.9 (10. 1).

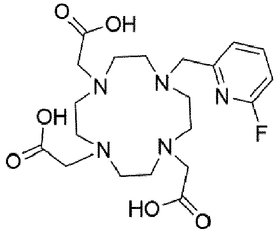

**Synthesis of ligand L⁶** (2,2',2"-(10-((6-fluoropyridin-2-yl)methyl)-1,4,7,10-tetraazacyclododecane-1,4,7-triyl)triacetic acid): Solution **2-(chloromethyl)-*6*-fluoropyridine** (81mg, 0.55 mmol, 1.1 equiv.) in MeCN (1 mL) was added to a solution of ***t*-BuDO3A**·HBr (300 mg, 0.50 mmol, 1.0 equiv.) in MeCN (10 mL) followed by addition of dried **K₂CO₃** (278 mg, 2.02 mmol, 4 equiv.) and the resulting suspension was stirred for 16 hours at RT. The solids were filtered off and the filtrate was concentrated on rotary evaporator. Resulting oil was purified on preparative HPLC (C18, MeCN/H₂O gradient with 0.1 % FA in the mobile phase). Fractions containing pure product in the form of *tert*-butyl ester were pooled, evaporated to dryness and several times co-evaporated with MeOH to remove MeCN. The residue was dissolved in neat **TFA** (7 mL) and stirred for 16 h at RT. Volatiles were removed on rotary evaporator and the residue was purified on preparative HPLC (C18, MeCN/H₂O gradient with 0.1 % FA in the mobile phase). Fractions with product were joined and lyophilized to give product in the form of FA/TFA salt as white solid. **Yield:** 210 mg (73 %, 2 steps, based on ***t*-BuDO3A**·HBr). **NMR** (D₂O): **¹H** δ_{H} 3.01-3.65 (*mc*, C*H*₂-COOH, m, 22H); 3.98 (C*H*₂-arom, s, 2H); 7.06 *(arom,* m, 1H); 7.58 *(arom,* m, 1H); 7.96 *(arom,* m, 1H); **¹³C{¹H}** δ_{C} 48.8 *(mc,* s); 48.9 *(mc,* s); 51.2 *(mc,* s); 51.8 *(mc,* s); 54.5 (*C*H₂-COOH, s); 56.8 (*C*H₂-COOH, s); 58.0 (*C*H₂-arom, s); 109.7 *(arom,* d, ²*J*_{CF} = 35 Hz ); 123.0 *(arom,* d, ⁴*J*_{CF} = 4 Hz); 144.7 *(arom,* d, ³*J*_{CF} = 8 Hz); 154.8 *(arom,* s); 163.3 *(arom,* d, ¹*J*_{CF} = 239 Hz); 170.2 (*C*O, s); 174.5 (*C*O, s). **¹⁹F{¹H}** *δ*_{F} -82.1 (s). **ESI-HRMS:** 478.2069 [M+Na]⁺ (theor. [C₂₀H₃₀N₅O₆F₁Na₁]⁺ = 478.2072). **EA** (C₂₀H₃₀N₅O₆F₁·0.5TFA·1.4FA, *M*_{R} = 576.9): C 46.6 (46.9); H 5.8 (5.8); N 12.1 (12.4); F 8.2 (8.0).

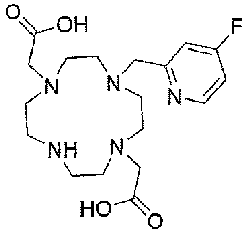

**Synthesis of ligand L⁷** (2,2'-(4-((4-fluoropyridin-2-yl)methyl)-1,4,7,10-tetraazacyclododecane-1,7-diyl)diacetic acid): Solution of 4 (127 mg, 0.87 mmol, 1.00 equiv.) in MeCN (1 mL) was added to a solution of ***t*-BuDO2A** (350 mg, 0.87 mmol, 1.0 equiv.) in MeCN (44 mL) followed by addition of dried **K₂CO₃** (121 mg, 0.87 mmol, 1 equiv.) and the resulting suspension was stirred for 24 hours at RT. The solids were filtered off and the filtrate was concentrated on rotary evaporator. Resulting oil was purified on preparative HPLC (C18, MeCN/H₂O gradient with 0.1 % TFA in the mobile phase). Fractions containing pure product in the form of tert-butyl ester were pooled, evaporated to dryness and several times co-evaporated with MeOH to remove MeCN. The residue was dissolved in neat **TFA** (5 mL) and stirred for 16 h at RT. Volatiles were removed on rotary evaporator and the residue was purified on preparative HPLC (C18, MeCN/H₂O gradient with 0.1 % TFA in the mobile phase). Fractions with product were joined and lyophilized to give product in the form of TFA salt as white solid. **Yield:** 206 mg (36 %, 2 steps, based on ***t*-BuDO2A**). **NMR** (D₂O): **¹H** δ_{H} 2.96-3.60 (*mc*, C*H*₂-COOH, m, 20H); 4.70 (C*H*₂-arom, s, 2H); 7.29 *(arom,* ddd, 1H, ³*J*_{HF} = 8 Hz, ³*J*_{HH} = 6 Hz, ⁴*J*_{HH} = 3 Hz); 7.34 *(arom,* dd, 1H, ³*J*_{HF} = 9 Hz, ⁴*J*_{HH} = 3 Hz); 8.51 *(arom,* dd, 1H, ⁴*J*_{HF} = 8 Hz, ³*J*_{HH} = 6 Hz); **¹³C{¹H}** δ_{C} 43.0 *(mc,* s); 49.2 *(mc,* s); 50.0 *(mc,* s); 52.4 (*mc*, s); 54.5 (*C*H₂-COOH, s); 57.8 (*C*H₂-arom, d, ⁴*J*_{CF} = 3 Hz); 112.5 *(arom,* d, ²*J*_{CF} = 19 Hz ); 113.5 *(arom,* d, ²*J*_{CF} = 17 Hz); 152.6 *(arom,* d, ³*J*_{CF} = 8 Hz); 153.0 *(arom,* d, ³*J*_{CF} = 8 Hz); 170.3 *(arom,* d, ¹*J*_{CF} = 264 Hz); 174.9 (*C*O, s). **¹⁹F{¹H}** *δ*_{F} -100.1 (s). **ESI-HRMS:** 398.2197 [M+H]⁺ (theor. [C₁₈H₂₉N₅O₄F₁]⁺ = 398.2198). EA (C₁₈H₂₈F₁N₅O₄·2.2TFA·0.9H₂O, *M*_{R} = 664.5): C 40.0 (39.9); H 4.4 (4.5); N 9.3 (9.1); F 22.1 (22.2).

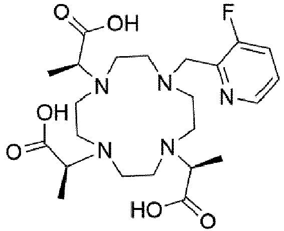

**Synthesis of ligand L⁸** ((2S,2'S,2"S)-2,2',2"-(10-((3-fluoropyridin-2-yl)methyl)-1,4,7,10-tetraazacyclododecane-1,4,7-triyl)tripropionic acid): Solution of **3** (13 mg, 92.2 µmol, 1.5 equiv.)) was added to a solution of 6 (45 mg, 61.7 µmol, 1.0 equiv.) in MeCN (1.2 mL) followed by addition of dried **K₂CO₃** (43 mg, 307 µmol, 5 equiv.) and the resulting suspension was stirred for 16 hours at RT. The solids were filtered off and the filtrate was concentrated on rotary evaporator. Resulting oil was purified on preparative HPLC (C18, MeCN/H₂O gradient with 0.1 % FA in the mobile phase). Fractions containing pure product in the form of *tert*-butyl ester were pooled, evaporated to dryness and several times co-evaporated with MeOH to remove MeCN. The residue was dissolved in MeOH (3 mL) followed by addition of **Pd@C** catalyst (10%, 10 mg). The flask was briefly washed with argon and then **H₂** gas (from balloon) was introduced. After 30 min, the catalyst was filtered off and the mixture was evaporated to dryness. The residue was purified on preparative HPLC (C18, MeCN/H₂O gradient with 0.1 % TFA in the mobile phase). Fractions with product were joined and lyophilized to give product in the form of TFA salt as white solid. **Yield:** 4.2 mg (13 %, 2 steps, based on 6). **NMR** (D₂O): **¹H** δ_{H} 1.26-1.30 (C*H*₃, m, 3H); δ_{H} 1.49 (C*H*₃, bs, 6H); δ_{H} 2.70-3.95 (*mc*, C*H*₂-COOH, m, 19H); 4.23-4.30 (C*H*₂-arom, m, 2H); 7.60 *(arom,* m, 1H); 7.84 *(arom,* m, 1H); 8.47 *(arom,* m, 1H); **¹³C{¹H}** δ_{C} 8.6 (*C*H₃, s); 10.3 (*C*H₃, s); 47.7, 47.8 (*mc*); 50.7 (*C*H₂-arom, s); 56.4 (*C*CH₃H-COOH, s); 61.9 (*C*CH₃H-COOH, s); 126.6 *(arom,* d, ³*J*_{CF} = 6 Hz ); 127.4 *(arom,* d, ²*J*_{CF} = 20 Hz); 142.8 *(arom,* d, ²*J*_{CF} = 19 Hz); 144.7 *(arom,* d, ⁴*J*_{CF} = 5 Hz); 159.1 *(arom,* d, ¹*J*_{CF} = 254 Hz); 177.2 (CO, s). **¹⁹F{¹H}** *δ*_{F} -123.2 (s). **ESI-HRMS:** 498.2719 [M+H]⁺ (theor. [C₂₃H₃₇N₅O₆F₁]⁺ = 498.2722). **EA** (C₂₃H₃₆N₅O₆F₁·0.1TFA·0.6H₂O, *M*_{R} = 519.8): C 53.0 (53.4); H 7.2 (7.0); N 13.5 (13.4); F 4.8 (4.6).

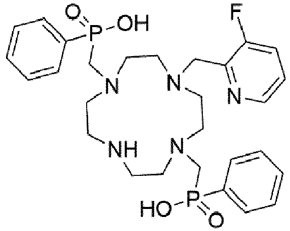

**Synthesis of ligand L⁹** (((4-((3-fluoropyridin-2-yl)methyl)-1,4,7,10-tetraazacyclododecane-1,7-diyl)bis(methylene))bis(phenylphosphinic acid)): Solution of 3 (6 mg, 40.0 µmol, 0.5 equiv.)) was added to a solution of **8** (41 mg, 80.6 µmol, 1.0 equiv.) in MeCN (1 mL) followed by addition of dried **K₂CO₃** (11 mg, 80.6 µmol, 1 equiv.) and the resulting suspension was stirred for 16 hours at RT. The solids were filtered off and the filtrate was concentrated on rotary evaporator. Resulting oil was purified on preparative HPLC (C18, MeCN/H₂O gradient with 0.1 % FA in the mobile phase). Fractions containing pure product in the form of methyl ester were pooled, evaporated to dryness and several times co-evaporated with MeOH to remove MeCN. The residue was dissolved in mixture of MeOH (1 mL) and H₂O (0.2 mL) followed by addition of **1M NaOH** (2 mL). The reaction mixture was neutralized with formic acid after 4 days of stirring at RT and the mixture was evaporated to dryness. The residue was purified on preparative HPLC (C18, MeCN/H₂O gradient with 0.1 % TFA in the mobile phase). Fractions with product were joined and lyophilized to give product in the form of white solid. **Yield:** 9.1 mg (18 %, 2 steps, based on **8**). **NMR** (D₂O): **¹H** δ_{H} 2.97-3.24 (me, C*H₂*-PhPOOH m, 20H); 4.54 (C*H*₂-arom, d, 2H, ⁵*J*_{HF} = 2 Hz,); 7.46-7.81 *(arom,* m, 12H); 8.42-8.47 *(arom,* m, 1H); **¹³C{¹H}** δ_{C} 44.0 *(mc),* 50.3, 51.6, 51.9, 54.3, 55.1 (*mc*, *C*H₂-PhPOOH); 52.4 (*C*H₂-arom, s); 125.9 *(arom,* d, ²*J*_{CF} = 19 Hz ); 127.9 *(arom,* d, ³*J*_{CF} = 5 Hz); 129.2 *(arom,* d, ²*J*_{CP} = 11 Hz); 131.2 *(arom,* d, ²*J*_{CP} = 10 Hz); 131.9 *(arom,* d, ⁴*J*_{CP} = 3 Hz); 137.5 *(arom,* d, ²*J*_{CF} = 15 Hz); 137.6 *(arom,* d, ¹*J*_{CP} = 60 Hz); 146.4 *(arom,* d, ⁴*J*_{CF} = 5 Hz); 159.4 *(arom,* d, ¹*J*_{CF} = 258 Hz); 177.2 (CO, s). **¹⁹F{¹H}** *δ*_{F} -122.9 (s); **³¹P{¹H}** δ 30.05. **ESI-HRMS:** 590.2454 [M+H]⁺ (theor. [C₂₈H₃₉N₅O₄F₁P_{2]}⁺ = 590.2456). **EA** (C₂₈H₃₈N₅O₄F₁P₂·0.1TFA·0.8H₂O, *M*_{R} = 615.4): C 55.0 (55.0); H 6.5 (6.3); N 4.0 (4.1); F 11.4 (11.2).

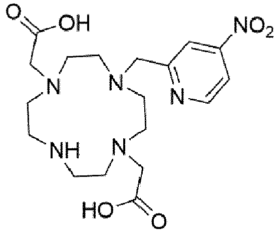

**Synthesis of ligand L¹⁰** (2,2'-(4-((4-nitropyridin-2-yl)methyl)-1,4,7,10-tetraazacyclododecane-1,7-diyl)diacetic acid): Solution of **1** (376 mg, 2.18 mmol, 1.0 equiv.) was added to a solution of ***t*-BuDO2A** (873 mg, 2.18 mmol, 1.0 equiv.) in MeCN (109 mL) followed by addition of dried **K₂CO₃** (301 mg, 2.18 mmol, 1.0 equiv.) and the resulting suspension was stirred for 39 hours at RT. The solids were filtered off and the filtrate was concentrated on rotary evaporator. Resulting oil was purified on preparative HPLC (C18, MeCN/H₂O gradient with 0.1 % TFA in the mobile phase). Fractions containing pure product in the form of tert-butyl ester were pooled, evaporated to dryness and several times co-evaporated with MeOH to remove MeCN. The residue was dissolved in neat **TFA** (6 mL) and stirred for 14 h at RT. Volatiles were removed on rotary evaporator and the residue was purified on preparative HPLC (C18, MeCN/H₂O gradient with 0.1 % TFA in the mobile phase). Fractions with product were joined and lyophilized to give product in the form of TFA salt as yellowish solid. **Yield:** 358 mg (26 %, 2 steps, based on ***t*-BuDO2A**). **NMR** (D₂O): **¹H** δ_{H} 2.88-3.74 (*mc,* C*H*₂-COOH, m, 20H); 4.91 (C*H*₂-arom, s, 2H); 8.21 *(arom,* dd, 1H, ³*J*_{HH} = 5 Hz, ⁴*J*_{HH} = 2 Hz); 8.27 *(arom,* dd, 1H, ³*J*_{HH} = 2 Hz, ⁵*J*_{HH} = 1 Hz); 8.85 (*arom,* dd, 1H, ³*J*_{HH} = 5 Hz, ⁵*J*_{HH} = 1 Hz **¹³C{¹H}** δ_{C} 43.0 *(mc,* s); 49.1 *(mc,* s); 50.1 *(mc,* s); 52.7 *(mc,* s); 54.5 (*C*H₂-COOH, s); 57.8 (*C*H₂-arom, s); 117.3 *(arom,* s); 118.1 *(arom,* s); 152.5 *(arom,* s); 153.1 *(arom,* s); 175.0 (*C*O, s). **ESI-HRMS:** 425.2143 [M+H]⁺ (theor. [C₁₈H₂₉N₆O₆]⁺ = 425.2143). **EA** (C₁₈H₂₈N₆O₆·1.7TFA·1.4H₂O, *M*_{R} = 643.5): C 39.9 (39.8); H 5.1 (4.4); N 13.1 (12.4); F 15.1 (14.8).

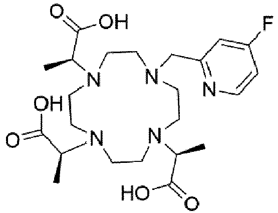

**Synthesis of ligand L¹¹** ((2*S*,2'*S*,2"*S*)-2,2',2"-(10-((4-fluoropyridin-2-yl)methyl)-1,4,7,10-tetraazacyclododecane-1,4,7 triyl)tripropionic acid): Intermediate 9 (18.7 mg, 91.2 µmol, 1.5 equiv.)) was mixed with intermediate 6 (44.5 mg, 60.8 µmol, 1.0 equiv.) in tBuOH (2.4 mL) followed by addition of **DIPEA** (53 µl, 304 µmol, 5 equiv.) and the resulting solution was stirred for 16 hours at 80°C. The liquids were evaporated on rotary evaporator and resulting oil was purified on preparative HPLC (C18, MeCN/H₂O gradient with 0.1 % FA in the mobile phase). Fractions containing pure product in the form of benzyl ester were pooled, evaporated to dryness and several times co-evaporated with MeOH to remove MeCN. The residue was dissolved in MeOH (3 mL) followed by addition **AcOH** (14 µl, 246 µmol, 4 eqiv.) and **Pd@C** catalyst (10%, 5 mg). The flask was briefly washed with argon and then **Hz** gas (from balloon) was introduced. After 30 min, the catalyst was filtered off and the mixture was evaporated to dryness. The residue was purified on preparative HPLC (C18, MeCN/H₂O gradient with 0.1 % TFA in the mobile phase). Fractions with product were joined and lyophilized to give product in the form of TFA salt as white solid. **Yield:** 4.1 mg (13 %, 2 steps, based on 6 assuming L¹¹·0.1TFA). **NMR** (DzO, ref. tBuOH): **¹H** δ_{H} 1.11 (C*H*₃, d, 3H, ³*J*_{HH} = 7 Hz); 1.22 (C*H*₃, d, 3H, ³*J*_{HH} = 7 Hz); 1.46 (C*H*₃, d, 3H, ³*J*_{HH} = 7 Hz,); 2.49-2.56 *(mc,* m, 2H); 2.70 (*mc*, m, 1H); 2.82-3.16 (*mc*, m, 9H); 3.27-3.46 (*mc*, m, 5H); 3.77 (C*H*CH₃COOH, q, 1H, ³*J*_{HH} = 7 Hz ); 4.07 (C*H*CH₃COOH, q, 1H, ³*J*_{HH} = 8 Hz); 4.13 (m, 1H); 4.50 (C*H*₂-arom, d, 1H, ²*J*_{HH} = 13 Hz); 7.10 *(arom,* ddd, 1H, ³*J*_{HF} = 8 Hz, ³*J*_{HH} = 6 Hz, ⁴*J*_{HH} = 3 Hz); 7.60 *(arom,* dd, 1H, ³*J*_{HF} = 8 Hz, ⁴*J*_{HH} = 3 Hz);; 8.36 *(arom,* m, 1H); **¹³C{¹H}** δ_{C} 9.0 (*C*H₃, s); 10.5 (*C*H₃, s); 11.0 (*C*H₃, s); 44.0, 46.4, 47.1, 47.4, 47.8, 48.2, 49.0 (*mc*, s); 58.1 (*C*H₂-arom, s); 57.4 (*C*CH₃H-COOH, s); 61.3 (*C*CH₃H-COOH, s); 62.3 (*C*CH₃H-COOH, s); 112.8 *(arom,* d, ²*J*_{CF} = 18 Hz ); 115.1 *(arom,* d, ²*J*_{CF} = 18 Hz); 150.9 *(arom,* d, ³*J*_{CF} = 8 Hz); 160.0 *(arom,* s); 170.8 *(arom,* d, ¹*J*_{CF} = 266 Hz); 172.0 (*C*O, s); 174.9 (*C*O, s); 180.2 (*C*O, s). **¹⁹F{¹H}** *δ*_{F} - 96.4 (s). **ESI-HRMS:** 498.2724 [M+H]⁺ (theor. [C₂₃H₃₇N₅O₆F₁]⁺ = 498.2722).

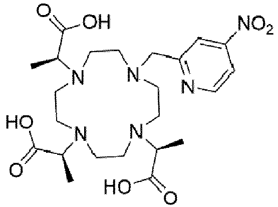

**Synthesis of ligand L¹²** ((2*S*,2'*S*,2"*S*)-2,2',2"-(10-((4-nitropyridin-2-yl)methyl)-1,4,7,10-tetraazacyclododecane-1,4,7-triyl)tripropionic acid): Intermediate 1 (8.2 mg, 47.6 µmol, 1.6 equiv.)) was mixed with intermediate **13** (15.0 mg, 28.9 µmol, 1.0 equiv.) in tBuOH (1.3 mL) followed by addition of **DIPEA** (28 µl, 158 µmol, 5.5 equiv.) and the resulting solution was stirred for 16 hours at 80°C. The liquides were evaporated on rotary evaporator and resulting oil was purified on preparative HPLC (C18, MeCN/H₂O gradient with 0.1 % FA in the mobile phase). Fractions containing pure product in the form of ethyl ester were pooled, evaporated to dryness and several times co-evaporated with MeOH to remove MeCN. The residue was dissolved in mixture of MeCN (500 µl) and H₂O (330 µl) followed by addition of 1M solution of **LiOH** (296 µl, 296 µmol, 10 equiv.) After 47 hours, the reaction mixture was quenched with **FA** (10 µl, 220 µmol, 7.6 equiv.) and the mixture was evaporated to dryness. The residue was purified on preparative HPLC (C18, MeCN/H₂O gradient with 0.1 % FA in the mobile phase). Fractions with product were joined and lyophilized to give product in the form of FA salt as yellowish solid. **Yield:** 6.0 mg (33 %, 2 steps, based on **13). NMR** (DzO, ref. tBuOH): **¹H** δ_{H} 1.24 (C*H*₃, d, 3H, ³*J*_{HH} = 7 Hz); 1.25 (C*H*₃, d, 3H, ³*J*_{HH} = 7 Hz); 1.59 (C*H*₃, d, 3H, ³*J*_{HH} = 7 Hz); 2.60-2.64 *(mc,* m, 2H); 2.83-3.05 (*mc*, C*H*CH₃COOH, m, 6H); 3.10-3.31 (*mc*, m, 5H); 3.41-3.55 (*mc*, CHz-arom, m, 4H); 3.98 (C*H*CH₃COOH, q, 1H, ³*J*_{HH} = 7 Hz ); 4.20 (C*H*CH₃COOH, q, 1H, ³*J*_{HH} = 8 Hz); 4.27 (m, 1H); 4.78 (C*H*₂-arom, m, 1H); 8.07 *(arom,* dd, 1H, ³*J*_{HH} = 6 Hz, ⁴*J*_{HH} = 2 Hz); 8.52 *(arom,* d, 1H, ⁴*J*_{HH} = 2 Hz); 8.75 *(arom,* d, 1H, ³*J*_{HH} = 6 Hz); **¹³C{¹H}** δ_{C} 8.0 (*C*H₃, s); 10.0 (*C*H₃, s); 10.7 (*C*H₃, s); 43.5, 46.1, 46.9, 47.4, 47.5, 47.8, 48.1, 49.2 (*mc*, s); 55.7 (*C*CH₃H-COOH, s); 58.2 (*C*H₂-arom, s); 60.9 (*C*CH₃H-COOH, s); 62.1 (*C*CH₃H-COOH, s); 117.2 *(arom);* 120.1 *(arom);* 151. *(arom);* 156.0 *(arom,* s); 160.3 *(arom);* 171.6 (CO, s); 174.7 (*C*O, s); 177.1 (*C*O, s). **ESI-HRMS:** 525.2667 [M+H]⁺ (theor. [C₂₃H₃₇N₆O₈]⁺ = 525.2667). **EA** (C₂₃H₃₆N₆O₈·2.2FA·0.6H₂O, *M*_{R} = 627.4): C 47.5 (47.6); H 6.6 (6.7); N 13.2 (13.1).

### Example 3: synthesis of metal chelates

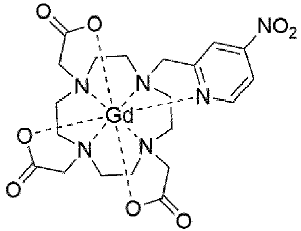

**Synthesis of complex [Gd(L¹)]**: In a glass vial (25 mL), **L¹**·1.8TFA·1.0H₂O (138 mg, 0.20 mmol, 1.0 equiv.) was dissolved in aq. **MOPS/NaOH buffer** (3M, pH 7.0, 2 mL, 6 mmol, 30 equiv.) followed by addition of aq. **GdCl₃** (100 mM, 2.1 mL, 0.21 mmol, 1.05 equiv.) and the resulting solution was stirred at RT for 15 mins. The mixture was then purified by preparative HPLC (C18, H₂O/MeCN gradient with 0.1% FA additive). Fractions with product were joined and lyophilized to give product as a white solid with yellow tint. **Yield:** 111 mg (81%, 1 step, based on **L¹**·1.8TFA·1.0H₂O). **ESI-HRMS:** 638.1203 [M+H]⁺ (theor. [C₂₀H₂₈N₆O₈Gd]⁺ = 638.1204). **EA** (C₂₀H₂₇N₆O₈Gd·2.5H₂O, *M*_{R} = 687.0): C 35.0 (35.4); H 4.7 (4.4); N 12.2 (11.9); Gd 22.9 (19.4).

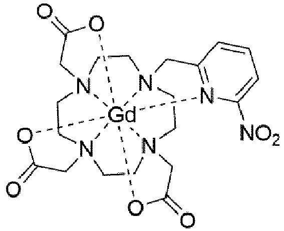

**Synthesis of complex [Gd(L²)]**: In a glass vial (4 mL), **L²**·0.8FA·0.2H₂O (37.4 mg, 71.5 µmol, 1.0 equiv.) was dissolved in aq. **MOPS/NaOH buffer** (3M, pH 7.0, 0.95 mL, 2.85 mmol, 40 equiv.) followed by addition of aq. **GdCl₃** (100 mM, 0.78 mL, 78 µmol, 1.1 equiv.) and the resulting solution was stirred at RT for 1 hour. The mixture was then purified by preparative HPLC (C18, H₂O/MeCN gradient with 0.1% FA additive). Fractions with product were joined and lyophilized to give product as a solid with yellow tint. **Yield:** 33 mg (62 %, 1 step, based on **L²**·0.8FA·0.2H₂O). **ESI-HRMS:** 638.1201 [M+H]⁺ (theor. [C₂₀H₂₈N_{O}0₈Gd]⁺ = 638.1204). EA (C₂₀H₂₇N₆O₈Gd·2.1FA·1.4H₂O, *M*_{R} = 758.6): C 35.0 (34.9); H 4.5 (4.4); N 11.1 (11.2); Gd 20.7 (20.8).

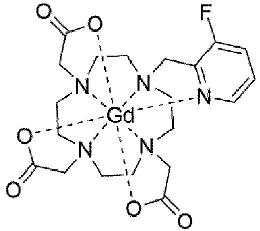

**Synthesis of complex [Gd(L³)]**: In a glass vial (25 mL), **L³**·0.7TFA·1.1FA (100 mg, 0.17 mmol, 1.0 equiv.) was dissolved in aq. **MOPS/NaOH buffer** (3M, pH 7.0, 1.7 mL, 5.1 mmol, 30 equiv.) followed by addition of aq. **GdCl₃** (100 mM, 1.9 mL, 0.19 mmol, 1.1 equiv.) and the resulting solution was stirred at RT for 1 hour. The mixture was then purified by preparative HPLC (C18, H₂O/MeCN gradient with 0.1% FA additive). Fractions with product were joined and lyophilized to give product as white solid. **Yield:** 98 mg (88 %, 1 step, based on **L³**·0.7TFA·1.1FA). **ESI-HRMS:** 611.1257 [M+H]⁺ (theor. [C₂₀H₂₈N₅O₆Gd₁F₁]⁺ = 611.1259). EA (C₂₀H₂₇N₅O₆Gd₁F₁·2.5H₂O, *M*_{R} = 654.7): C 36.7 (37.2); H 4.9 (4.9); N 10.7 (10.6); Gd 24.0 (21.4); F 2.9 (2.6).

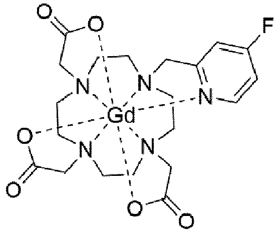

**Synthesis of complex [Gd(L⁴)]:** In a glass vial (25 mL), **L⁴**·2.6TFA·0.3H₂O (156 mg, 0.21 mmol, 1.0 equiv.) was dissolved in aq. **MOPS/NaOH buffer** (3M, pH 7.0, 1.9 mL, 5.8 mmol, 28 equiv.) followed by addition of aq. **GdCl₃** (100 mM, 2.16 mL, 0.22 mmol, 1.05 equiv.) and the resulting solution was stirred at RT for 15 mins. The mixture was then purified by preparative HPLC (C18, HzO/MeCN gradient with 0.1% FA additive). Fractions with product were joined and lyophilized to give product as white solid. **Yield:** 82 mg (59 %, 1 step, based on **L⁴**·2.6TFA·0.3H₂O). **ESI-HRMS:** 611.1275 [M+H]⁺ (theor. [C₂₀H₂₈N₅O₆Gd₁F₁]⁺ = 611.1259). EA (C₂₀H₂₇N₅O₆Gd₁F₁·2.5H₂O, *M*_{R} = 654.7): C 36.7 (36.2); H 4.9 (5.0); N 10.7 (10.5); Gd 24.0 (19.8); F 2.9 (2.8).

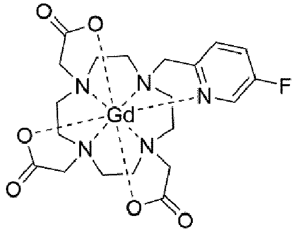

**Synthesis of complex [Gd(L⁵)]**: In a glass vial (25 mL), **L⁵**·0.7TFA·1.3FA (100 mg, 0.17 mmol, 1.0 equiv.) was dissolved in aq. **MOPS/NaOH buffer** (3M, pH 7.0, 1.7 mL, 5.1 mmol, 30 equiv.) followed by addition of aq. **GdCl₃** (100 mM, 1.9 mL, 0.19 mmol, 1.1 equiv.) and the resulting solution was stirred at RT for 1 hour. The mixture was then purified by preparative HPLC (C18, H₂O/MeCN gradient with 0.1% FA additive). Fractions with product were joined and lyophilized to give product as white solid. **Yield:** 95 mg (82 %, 1 step, based on **L**⁵·0.7TFA·1.3FA). **ESI-HRMS:** 611.1257 [M+H]⁺ (theor. [C₂₀H₂₈N₅O₆Gd₁F₁]⁺ = 611.1259). **EA** (C₂₀H₂₇N₅O₆Gd₁F₁·4H₂O, *M*_{R} = 681.8): C 35.2 (35.4); H 5.2 (5.0); N 10.3 (10.1); Gd 23.1 (20.8); F 2.8 (2.4).

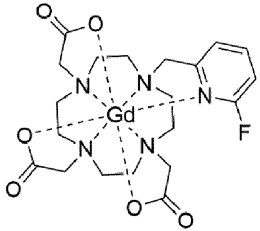

**Synthesis of complex [Gd(L⁶)]**: In a glass vial (25 mL), **L⁶**·0.5TFA·1.4FA (150 mg, 0.26 mmol, 1.0 equiv.) was dissolved in aq. **MOPS/NaOH buffer** (3M, pH 7.0, 2.6 mL, 7.8 mmol, 30 equiv.) followed by addition of aq. **GdCl₃** (100 mM, 3.1 mL, 0.31 mmol, 1.2 equiv.) and the resulting solution was stirred at RT for 1 hour. The mixture was then purified by preparative HPLC (C18, H₂O/MeCN gradient with 0.1% FA additive). Fractions with product were joined and lyophilized to give product as white solid. **Yield:** 151 mg (86 %, 1 step, based on **L⁶**·0.5TFA·1.4FA). **ESI-HRMS:** 611.1256 [M+H]⁺ (theor. [C₂₀H₂₈N₅O₆Gd₁F₁]⁺ = 611.1259). **EA** (C₂₀H₂₇N₅O₆Gd₁F₁·1.5H₂O·0.8FA, *M*_{R} = 673.5): C 37.1 (37.3); H 4.7 (4.6); N 10.4 (10.7); Gd 23.4 (23.4); F 2.8 (2.5).

(C₂₀H₃₀N₅O₆F₁·0.5TFA-1.4FA, *M*_{R} = 576.9)

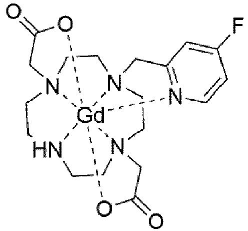

**Synthesis of complex [Gd(L⁷)]** : In a glass vial (25 mL), **L⁷**·2.2TFA·0.9H₂O (152 mg, 0.22 mmol, 1.0 equiv.) was dissolved in aq. **MOPS/NaOH buffer** (3M, pH 7.0, 1.64 mL, 4.9 mmol, 21 equiv.) followed by addition of aq. **GdCl₃** (100 mM, 2.34 mL, 0.23 mmol, 1.05 equiv.) and the resulting solution was stirred at RT for 15 mins. The mixture was then purified by preparative HPLC (C18, H₂O/MeCN gradient with 0.1% FA additive). Fractions with product were joined and lyophilized to give product as white solid. **Yield:** 116 mg (77 %, 1 step, based on **L⁷**·2.2TFA·0.9H₂O). **ESI-HRMS:** 553.1214 [M+H]⁺ (theor. [C₁₈H₂₆N₅O₄Gd₁F₁]⁺ = 553.1204). **EA** (C₁₈H₂₆N₅O₄Gd₁F₁·3.1H₂O, *M*_{R} = 681.2): C 33.9 (34.5); H 4.7 (4.5); N 10.3 (9.9); Gd 23.1 (19.6); F 7.8 (7.4).

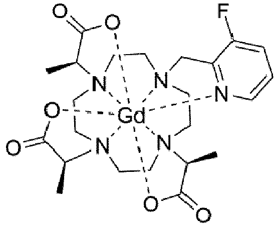

**Synthesis of complex [Gd(L⁸)]**: In a glass vial (25 mL), **L⁸**·0.1TFA·0.6H₂O (4 mg, 8.1 µmol, 1.0 equiv.) was dissolved in aq. **MOPS/NaOH buffer** (0.5M, pH 7.0, 1.00 mL, 0.5 mmol, 62 equiv.) followed by addition of aq. **GdCl₃** (100 mM, 97.0 µL, 9.7 µmol, 1.2 equiv.) and the resulting solution was stirred at RT for 1 hour. The mixture was then purified by preparative HPLC (C18, H₂O/MeCN gradient with 0.1% FA additive). Fractions with product were joined and lyophilized to give product as white solid. **Yield:** 4.6 mg. **ESI-HRMS:** 653.1728 [M+H]⁺ (theor. [C₂₃H₃₄N₅O₆Gd₁F₁]⁺ = 653.1729).

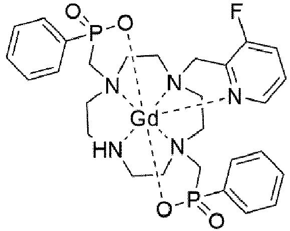

**Synthesis of complex [Gd(L⁹)]**: In a glass vial (25 mL), **L⁹**·0.1TFA·0.8H₂O (6 mg, 9.18 µmol, 1.0 equiv.) was dissolved in aq. **MOPS/NaOH buffer** (0.5M, pH 7.0, 1.00 mL, 0.5 mmol, 51 equiv.) followed by addition of aq. **GdCl₃** (100 mM, 117.0 µL, 11.7 µmol, 1.2 equiv.) and the resulting solution was stirred at RT for 20 hours. The mixture was then purified by preparative HPLC (C18, H₂O/MeCN gradient with 0.1% AcOH additive). Fractions with product were joined and lyophilized to give product as white solid. **Yield:** 5.4 mg. **ESI-HRMS:** 745.1461 [M+H]⁺ (theor. [C₂₈H₃₆N₅O₄Gd₁F₁P₂]⁺ = 745.1462).

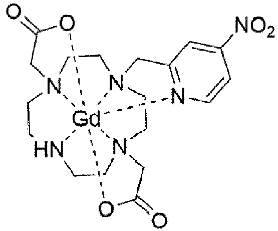

**Synthesis of complex [Gd(L¹⁰)]**: In a glass vial (25 mL), **L¹⁰**·1.7TFA·1.4H₂O (129 mg, 0.20 mmol, 1.0 equiv.) was dissolved in aq. **MOPS/NaOH buffer** (3M, pH 7.0, 2. mL, 8.02 mmol, 40 equiv.) followed by addition of aq. **GdCl₃** (100 mM, 2.2 mL, 0.22 mmol, 1.1 equiv.) and the resulting solution was stirred at RT for 1 hour. The mixture was then purified by preparative HPLC (C18, H₂O/MeCN gradient with 0.1% FA additive). Fractions with product were joined and lyophilized to give product as a solid with yellow tint. **Yield:** 92 mg (64 %, 1 step, based on **L¹⁰**·1.7TFA·1.4H₂O). **ESI-HRMS:** 580.1152 [M+H]⁺ (theor. [C₁₈H₂₆N₆O₆Gd₁]⁺ = 580.1149). EA (C₁₈H₂₆N₆O₆Gd₁·3FA, *M*_{R} = 717.8): C 35.1 (34.9); H 4.5 (4.1); N 11.7 (12.1); Gd 21.9 (19.9).

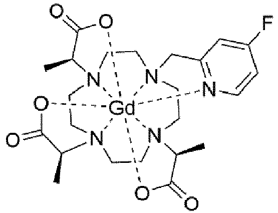

**Synthesis of complex [Gd(L¹¹)]**: In a glass vial (4 mL), **L¹¹**·0.1TFA (3.1 mg, 6.0 µmol, 1.0 equiv.) was dissolved in aq. **MOPS/NaOH buffer** (0.5M, pH 7.0, 0.738 mL, 369 µmol, 62 equiv.) followed by addition of aq. **GdCl₃** (100 mM, 72.0 µL, 7.2 µmol, 1.2 equiv.) and the resulting solution was stirred at RT for 1 hour. The mixture was then purified by preparative HPLC (C18, H₂O/MeCN gradient with 0.1% FA additive). Fractions with product were joined and lyophilized to give product as white solid. **Yield:** 3.5 mg. **ESI-HRMS:** 653.1731 [M+H]⁺ (theor. [C₂₃H₃₄N₅O₆Gd₁F₁]⁺ = 653.1729).

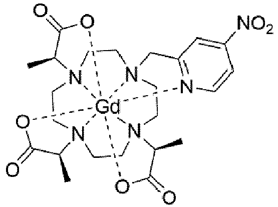

**Synthesis of complex [Gd(L¹²)]**: In a glass vial (4 mL), **L¹²**·2.2FA·0.6H₂O (3 mg, 4.8 µmol, 1.0 equiv.) was dissolved in aq. **MOPS/NaOH buffer** (0.5M, pH 7.0, 0.706 mL, 353 µmol, 73 equiv.) followed by addition of aq. **GdCl₃** (100 mM, 69.0 µL, 6.9 µmol, 1.4 equiv.) and the resulting solution was stirred at RT for 15 minutes. The mixture was then purified by preparative HPLC (C18, HzO/MeCN gradient with 0.1% FA additive). Fractions with product were joined and lyophilized to give product as white solid. **Yield:** 4.1 mg. **ESI-HRMS:** 680.1679 [M+H]⁺ (theor. [C₂₃H₃₄N₆O₈Gd₁]⁺ = 680.1674).

### Radiolabelling experiments

### Example 4: radiolabelling with ¹⁸F⁻ anion

### General procedure

A solution containing ¹⁸F⁻ produced on a cyclotron was absorbed on the QMA column (Sep-Pak, Waters) in a CO₃⁻ cycle and eluted with an eluting solution of 0.8 ml (8.3 mg TBAHCO₃ in a mixture of 0.4 ml H₂O and 0.4 ml MeCN). A volume containing the desired activity was transferred to a 4 ml glass vial with a stirring bar preheated for 120 °C and a stream of argon was introduced. The aqueous solution was evaporated and 300 µl of dry MeCN was introduced and evaporated again. This step was performed twice. 100 µl of 15 mM solution of the material for radiolabelling ([Gd(L¹)], [Gd(L⁴)] or L⁴) in a dry DMSO was introduced, followed by the 4 µl of 1 M solution of a TBAF in a THF. The vial was transferred to an aluminium heat block preheated to 90°C and the reaction mixture was stirred for 5 minutes. The reaction mixture was afterwards analysed on a HPLC equipped with the gamma and UV detector.

### Radiolabelling of [Gd(L¹)] by nucleophilic aromatic substitution

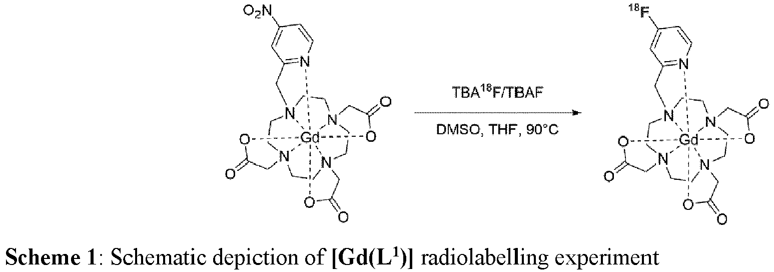

**Table 1: Radiolabelling experiments with [Gd(L¹)] as a precursor. All the reactions were done in dry solvents. The amount of precursor used was 1 mg.**

| Experiment number | Radiochemical yield (%) | Chemical conversion (%) |
|---|---|---|
| 1 | 11 | 78 |
| 2 | 17 | 76 |

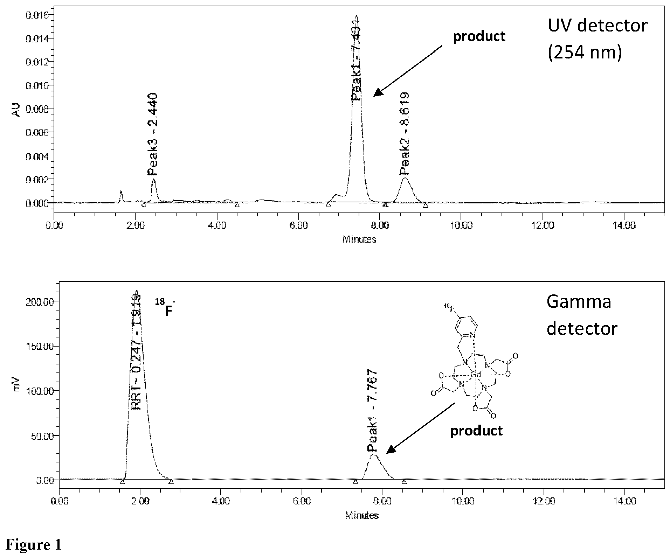

Chromatograms from this radiolabelling experiment are depicted in Figure 1. Radiochemical yield is defined as an amount of activity in the product expressed as the percentage of related starting activity.

### Radiolabelling of [Gd(L⁴)] by ¹⁸F<->¹⁹F isotopic exchange

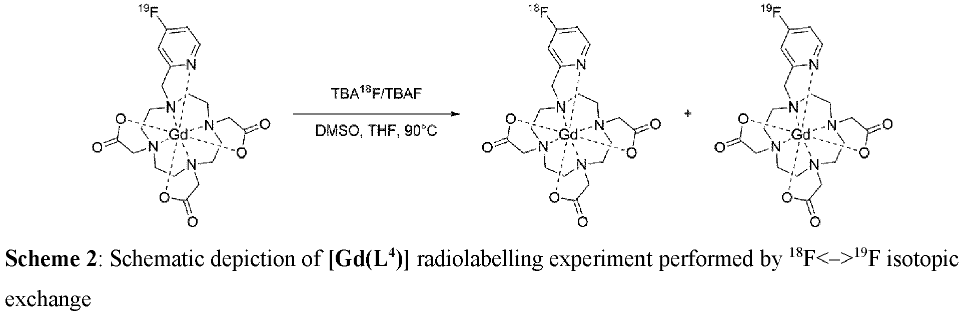

**Table 2: Radiolabelling experiments with [Gd(L⁴)] as a precursor. All the reactions were done in dry solvents. The amount of precursor used was 1 mg.**

| Experiment number | Radiochemical yield (%) | Chemical conversion (%) |
|---|---|---|
| 1 | 17 | 83 |
| 2 | 17 | 85 |
| 3 | 13 | 82 |

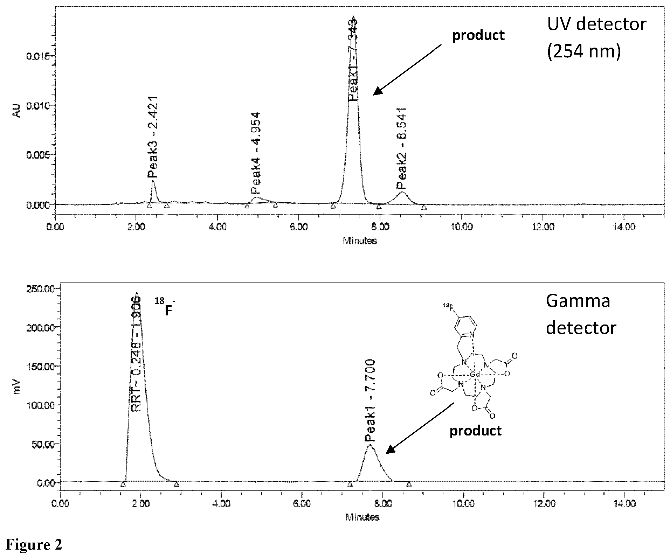

Chromatograms from this radiolabelling experiment are depicted in Figure 2.

### Radiolabelling of free ligand L⁴ by ¹⁸F<->¹⁹F isotopic exchange

The experiment with free ligand **L**⁴ was performed to demonstrate that the chelated metal is crucial for the radiolabelling reaction to proceed. The experiment was carried out using the same conditions as the other radiolabelling reactions.

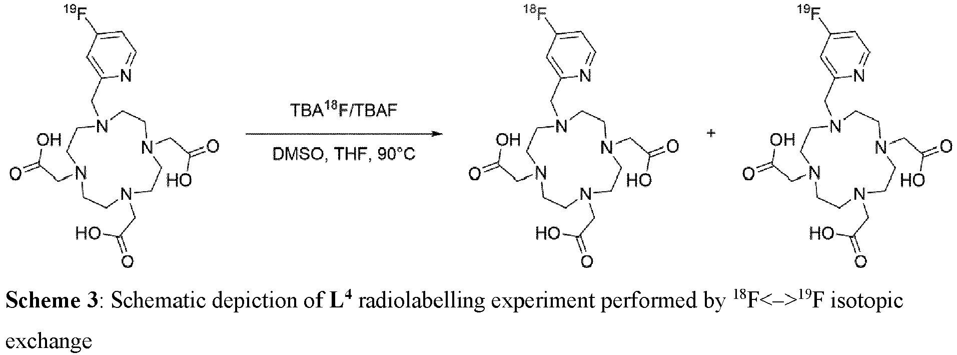

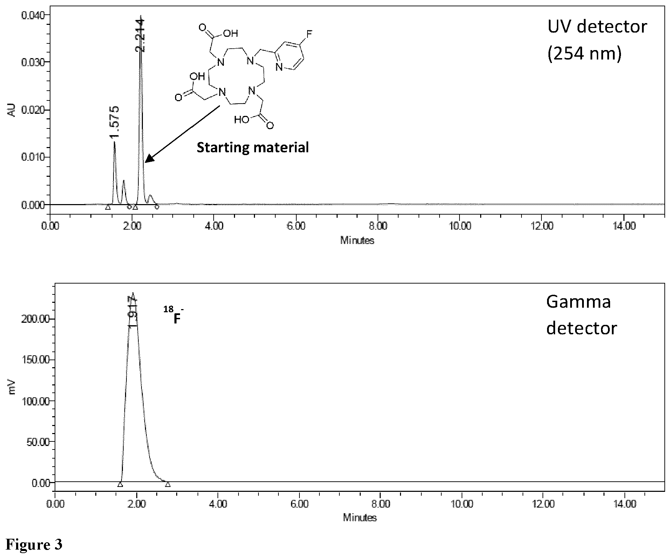

Chromatograms from this radiolabelling experiment are depicted in Figure 3.

**Table 3: Radiolabelling experiments with free ligand L⁴ as a precursor. All the reactions were done in dry solvents. The amount of precursor used was 1 mg.**

| Experiment number | Radiochemical yield (%) |
|---|---|
| 1 | 0 |
| 2 | 0 |

### Example 5: Relaxivity of [Gd(L⁴)] and [Gd(L⁷)]

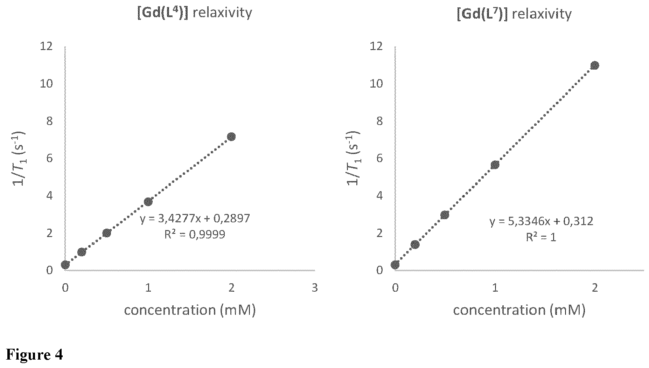

The value of relaxivity is directly proportional to the effectiveness of a compound as an MRI contrast agent. For the relaxivity determination, samples of **[Gd(L⁴)]** and **[Gd(L⁷)]** were prepared at 0, 0.2, 0.5, 1, 2 mM concentrations in a MOPS/NaOH buffer (50 mM, pH 7.0, 150 µL). The samples thus prepared were used for the determination of the longitudinal relaxation time *T*₁ values on the relaxometer at 37 °C and 0.47 T. From these values, it was possible to determine that the **[Gd(L⁴)]** has a relaxivity of 3.43 mM⁻¹s⁻¹ and **[Gd(L⁷)]** has a relaxivity of 5.33 mM⁻¹s⁻¹ (Table 4). The relaxivities were determined by linear regression of their concentration versus longitudal relaxation rate as can be seen in Figure 4. For comparison, the relaxivity of most clinically used MRI contrast agents at comparable conditions is within the range 3 - 4 mM⁻¹s⁻¹ (Rohrer, M.; Bauer, H.; Mintorovitch, J.; Requardt, M.; Weinmann, H.-J. Comparison of Magnetic Properties of MRI Contrast Media Solutions at Different Magnetic Field Strengths. Investigative Radiology 2005, 40 (11), 715-724. https://doi.org/10.1097/01.rli.0000184756.66360.d3).

**Table 4: Determination of the longitudinal relaxation time T₁ values of [Gd(L⁴)] and [Gd(L⁷)].**

| Compound | Relaxivity (mM⁻¹s⁻¹) |
|---|---|
| **[Gd(L4)]** | 3.43 |
| **[Gd(L⁷)]** | 5.33 |

### Example 6: Kinetic inertness of [Gd(L⁴)] and [Gd(L⁷)]

The kinetic inertness of **[Gd(L⁴)]** and **[Gd(L⁷)]** was tested using acid-assisted decomplexation with HCl (0.1M). Magnevist^{®}, gadolinium-DTPA (diethylenetriamine penta-acetic acid), a clinically used contrast agent was used as a reference and solution of GdCl₃ (1mM) in HCl (0.1M) was used as an example of fully released gadolinium from chelate. All measured complexes were used as 1 mM solutions with a volume of 150 µL and incubated at 37 °C. During decomplexation, the gadolinium ion detaches from the complex and becomes fully solvated. T₁ of the sample decreases during this process since solvated gadolinium shortens T₁ relaxation time to a greater degree than chelated gadolinium. The reaction reaches equilibrium when the complex is fully decomposed, and all the gadolinium is solvated. The *T*₁ relaxation time isn't decreasing in this equilibrium state anymore. The *T*₁ relaxation time values were determined on a relaxometer at 37 °C and a field strength of 0.47 T. The kinetic inertness was compared in paramagnetic relaxation rate: *R*₁ₚ = 1/ *T*₁ - 1/*T*_{1d}. The longitudinal relaxation time of the HCl (0.1M) corresponds to *T*_{1d}. A slow increase of *T*₁ values can be observed in the case of **[Gd(L⁴)]** during the measurement, which suggests that the complex is slowly decomposed. Over a 240-minute period, the *T*₁ value of **[Gd(L⁴)]** increased by 12 %. Situation is different in the case of Magnevist, where the reaction reaches its equilibrium already after 6 minutes (Table 5). The *R*₁ₚ values of the Magnevist sample didn't change over time, so it can be assumed that the complex fully decomposed. In the case of **[Gd(L⁷)]** the increase of *R*₁ₚ is slower in comparison to Magnevist, which suggest that the **[Gd(L⁷)]** is more kinetically inert. The measurements show that **[Gd(L⁴)]** and **[Gd(L⁷)]** are more kinetically inert than the clinically used Magnevist and thus suitable for *in vivo* applications. Graphs showing measurements of kinetic inertness are shown in Figure .

**Table 5: Comparison of kinetic inertness of [Gd(L⁴)], [Gd(L⁷)], Magnevist and solution of GdCl₃ by acid-assisted decomplexation in 0.1M HCl.**

| Time | 6 min | 8 min | 12 min | 4h | 24h |
|---|---|---|---|---|---|
| *R*₁ₚ of [Gd(L⁷)] (s⁻¹) | 7.2 | 7.9 | 8.8 | 9.5 | 9.5 |
| *R*₁ₚ of GdCl₃ (s⁻¹) | 9.0 | No data | No data | 9.1 | 9.0 |
| *R*₁ₚ of Magnevist(s⁻¹) | 8.8 | 8.7 | 8.7 | 8.8 | 8.7 |
| *R*₁ₚ of [Gd(L⁴)] (s⁻¹) | 3.4 | No data | No data | 3.8 | 5.3 |

## Claims

1. Cyclen based compound of general formula (I) 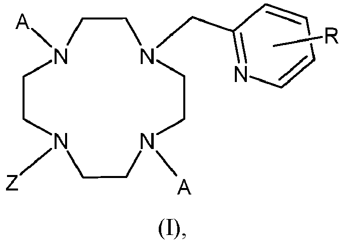 wherein
R is NO₂ or F;
A is independently selected from -CH₂COOH; -CH(CH₃)COOH; ; -CH((CH₂)ₙCH₃)COOH, wherein n is an integer in the range of from 1 to 3; and -CH₂P(=O)(OH)Ph;
Z is H or A;
for use in medicine and pharmacy, preferably for use for preparation of a multimodal PET/MRI contrast agent.

2. Cyclen based compound of general formula (I) for use according to claim 1, wherein A and Z are the same, preferably selected from -CH₂P(=O)(OH)Ph; and -CH(CH₃)COOH.

3. Cyclen based compound of general formula (I) for use according to claim 1, wherein A are the same, preferably selected from -CH₂P(=O)(OH)Ph; and -CH(CH₃)COOH; and Z is hydrogen.

4. Cyclen based compound of general formula (I) for use according to claim 1, wherein R is located in para-position in relation to the nitrogen atom of the pyridyl pendant arm.

5. Cyclen based compound of general formula (I) for use according to claim 1, which is selected from the group comprising:
2,2',2"-(10-((4-nitropyridin-2-yl)methyl)-1,4,7,10tetraazacyclododecane-1,4,7triyl)triacetic acid (**L**¹)
2,2',2"-(10-((6-nitropyridin-2-yl)methyl)-1,4,7,10tetraazacyclododecane-1,4,7triyl)triacetic acid (**L**²)
2,2',2"-(10-((3-fluoropyridin-2-yl)methyl)-1,4,7,10 tetraazacyclododecane-1,4,7 triyl)triacetic acid (**L**³)
2,2',2"-(10-((4-fluoropyridin-2-yl)methyl)-1,4,7,10 tetraazacyclododecane-1,4,7 triyl)triacetic acid (**L**⁴)
2,2',2"-(10-((5-fluoropyridin-2-yl)methyl)-1,4,7,10 tetraazacyclododecane-1,4,7 triyl)triacetic acid (**L**⁵)
2,2',2"-(10-((6-fluoropyridin-2-yl)methyl)-1,4,7,10 tetraazacyclododecane-1,4,7 triyl)triacetic acid (**L**⁶)
2,2'-(4-((4-fluoropyridin-2-yl)methyl)-1,4,7,10 tetraazacyclododecane-1,7-diyl)diacetic acid (**L**⁷)
(2*S*,2'*S*,2"*S*)-2,2',2"-(10-((3-fluoropyridin-2-yl)methyl)-1,4,7,10-tetraazacyclododecane-1,4,7-triyl)tripropionic acid (**L**⁸)
((4-((3-fluoropyridin-2-yl)methyl)-1,4,7,10 tetraazacyclododecane-1,7-diyl)bis(methylene))bis(phenylphosphinic acid) (**L**⁹)
2,2'-(4-((4-nitropyridin-2-yl)methyl)-1,4,7,10-tetraazacyclododecane-1,7-diyl)diacetic acid (**L**¹⁰)
(2*S*,2'*S*,2",*S*)-2,2',2"-(10-((4-fluoropyridin-2-yl)methyl)-1,4,7,10-tetraazacyclododecane-1,4,7-triyl)tripropionic acid (**L**¹¹)
(2S,2'S,2",S)-2,2',2"-(10-((4-nitropyridin-2-yl)methyl)-1,4,7,10-tetraazacyclododecane-1,4,7-triyl)tripropionic acid (**L**¹²)

6. Coordination compound of Gd(III) ion with the cyclen based compound of general formula (I) as defined in any one of the preceding claims 1 to 5, for use in medicine for preparation of a multimodal PET/MRI contrast agent.

7. Cyclen based compound of general formula (Ia) 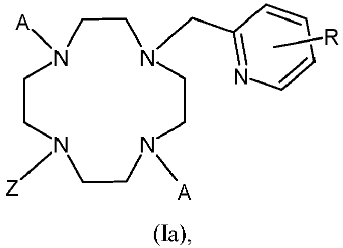 wherein
R is NO₂ or F; preferably R is F;
A is independently selected from -CH(CH₃)COOH; -CH((CH₂)ₙCH₃)COOH, wherein n is an integer in the range of from 1 to 3; and -CH₂P(=O)(OH)Ph; preferably A is -CH(CH₃)COOH;
Z is H or A, preferably Z is A.

8. Cyclen based compound of general formula (Ia) according to claim 7, wherein the R substituent is located in para-position in relation to the nitrogen atom of the pyridyl pendant arm.

9. Cyclen based compound of general formula (Ia) according to claim 7 or 8, wherein Z is A, and all A are -CH(CH₃)COOH of the same stereoconfiguration.

10. A multimodal PET/MRI contrast agent of general formula (VI) 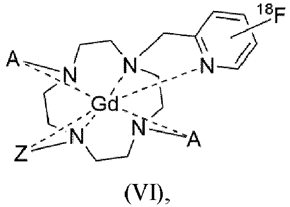
wherein A is independently selected from -CH₂COOH; -CH(CH₃)COOH; -CH((CH₂)ₙCH₃)COOH, wherein n is an integer in the range of from 1 to 3; and -CH₂P(=O)(OH)Ph;
Z is H or A;
preferably ¹⁸F is in para-position in relation to the nitrogen atom of the pyridyl pendant arm.

11. A method of synthesis of the multimodal PET/MRI contrast agent of general formula (VI) as defined in claim 10, comprising the following steps:
a) providing the cyclen based compound of general formula (I) as defined in claims 1 to 5;
b) reacting the cyclen based compound of general formula (I) with Gd³⁺ ion, thereby forming a Gd(III) chelate of the compound of general formula (I);
c) radiolabelling the Gd(III) chelate from step b) with ¹⁸F.

12. The method according to claim 11, wherein step c) is performed using ¹⁹F to ¹⁸F isotopic exchange on the pyridine moiety of the Gd(III) chelate, wherein R is F.

13. The method according to claim 11, wherein step c) is performed using nucleophilic aromatic substitution of nitro group with ¹⁸F on the pyridine moiety of the Gd(III) chelate, wherein R is nitro group.

14. A pharmaceutical formulation comprising the multimodal PET/MRI contrast agent of general formula (VI) according to claim 10, which further contains at least one conventionally used pharmaceutically acceptable auxiliary substance, preferably selected from the group containing solvents, buffers, ionization additives, antioxidants, antimicrobial additives.

15. The_multimodal PET/MRI contrast agent of general formula (VI) according to claim 10 and/or the pharmaceutical formulation according to claim 14, for use in medicine, preferably in multimodal PET/MRI diagnostic methods.
